(19) Europäisches Patentamt
European Patent Office
Office européen des brevets

(11) **EP 2 656 853 A1**

(12) **EUROPEAN PATENT APPLICATION**
published in accordance with Art. 153(4) EPC

(43) Date of publication:
30.10.2013 Bulletin 2013/44

(51) Int Cl.:
*A61K 35/30* (2006.01)     *A61K 35/48* (2006.01)
*A61K 38/00* (2006.01)     *A61P 25/00* (2006.01)
*A61P 9/10* (2006.01)

(21) Application number: 11851446.2

(22) Date of filing: 13.05.2011

(86) International application number:
PCT/RU2011/000327

(87) International publication number:
WO 2012/087180 (28.06.2012 Gazette 2012/26)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 24.12.2010 RU 2010152972

(71) Applicant: Zakrytoe Aktsionernoe Obschestvo
"Pharm-Sintez"
Moscow 111024 (RU)

(72) Inventors:
• NAZARENKO, Anna Borisovna
Moscow 121614 (RU)
• SOKOLOV, Mikhail Anatolevich
Moscow 107150 (RU)

(74) Representative: Held, Stephan et al
Meissner, Bolte & Partner GbR
Widenmayerstraße 47
80538 München (DE)

(54) **METHOD FOR TREATING ACUTE CEREBRAL AND SPINAL BLOOD FLOW DISORDERS OF AN ISCHAEMIC OR HAEMORRHAGIC NATURE**

(57)     The invention relates to the chemical and pharmaceutical industry and medicine; more specifically, it relates to the methods of treatment of the acute cerebral and spinal circulation accidents. The treatment method consists in administering the pharmaceutical composition that contains the biologically active substance which substance is the protein-polypeptide complex and the pharmaceutically acceptable excipients which excipients contain the pharmaceutically acceptable diluent which diluent contains the officinal buffer solution, high molecular weight substances, stabilizers, preservatives, and solubilizers. The pharmaceutical composition is administered, 0.05 to 0.8 mg/day, subcutaneously, intramuscularly, intravenously, intranasally, and/or intrathecally.

The protein-polypeptide complex, which is the biologically active substance (pharmacologic substance) of the pharmaceutical composition, is obtained from the mid first third to mid last third gestational age livestock embryonic brain tissue, which complex contains the negatively charged faintly acid, neutral proteins and polypeptides relating to the growth factor, differentiation factor, signaling molecules, and intercellular adhesion molecules, with the 5 to 200 kDa molecular masses, wherein at least 80% of the total protein mass has the 10 to 120 kDa molecular mass.

EP 2 656 853 A1

**Description**

**Pertinent Art**

The invention relates to the medicine, in particular, to the new ischemic and hemorrhagic character cerebral and spinal circulation accidents treatment method using the neuroprotective drugs.

The acute cerebrovascular accidents make an actual medico-social problem of the today's medicine, due to their high share in the population morbidity and mortality patterns, and high temporary labor incapacity and primary disablement indicants. In Russia, the mortality due to the cerebrovascular diseases, especially ischemic and hemorrhagic strokes, is on the second place in the total mortality pattern, not much lower than for the cardiac diseases. The stroke acute stage lethality is 35%; it grows by 12 to 15% to the end of the first year. The stroke-induced incapacitation is the major cause of the primary disablement. The stroke consequences consist in the damage to, or, often, loss, of the attacked brain region. This region becomes unable to perform its functions; hence, all kinds of the speech, consciousness, motion coordination, vision, and sensation disorders, and paralyses.

**Prior Art**

A number of the animal feedstock nootropic and neurometabolic activity protein-peptide nature drugs are known which drugs are used to treat the nervous system diseases. These preparations include:

1. Cerebrolysin: drug to treat the hemorrhagic or ischemic strokes, by Ebewe (Austria) (Encyclopedia of Drugs, 2006, p. 970) [1], a product of the intact porcine brain treatment;

2. Cerebrocurin: drug to treat the diseases associated with the central nervous system dysfunctions, by OOO "NIR" (Ukraine) (RF Patent # 2128511(1999) [2], a product of the animal embryonic brain treatment;

3. Cortexin: polypeptide nature drug obtained by extraction from the bovine and porcine brain cortex, by OOO "Geropharm" (Russia) (RF Patent # 2104702 (1999) [3], it has the nootropic, cerebroprotective, anticonvulsant, and antioxidant effects;

4. Cerebrolysate: drug improving the brain tissue resistance to the intoxication, hypoxia, hypoglycemia, and mechanical injury, by "Microgen" Scientific-Production Association, a federal state unitary enterprise ("Immunopreparat") (RF Patent # 2049472 (1999) [4]; it has the nootropic effect, and is a bovine brain cortex hydrolyzate; and

5. Semax (Met-Glu-His-Phe-Pro-Gly-Pro) : synthetic polypeptide, analog of ACTH/4-10/ fragment, with nootropic properties, and without hormonal activity (USSR Patent 939440, Class • 07 • 103/52, 1981) [5].

The Cerebrolysin contains the amino acids (~85%), low molecular weight peptides (15%), and microelements, the feedstock for which is the porcine brain. The neuroprotective and trophic effects of the drug are due to the specific peptides and amino acids, of max. 10, 000 Dalton molecular mass, where the alanine, leucine, and lysine dominate and determine the drug properties. The drug is intended for the intramuscular and intravenous administration, the biologically active components concentration is low; hence, the treatment is high dose and long term (Ed. Yu.F. Krylov. Register of Medicinal Agents of Russia. Moscow, Inpharmchim, 1993, p. 935). The heterogeneity of the component peptides (molecular masses, formulas, and properties) does not let to definitely associate the drug positive effects with any specific component. The Cerebrolysin action consists, first of all, in its predominant effect on the focal neurologic lesion intensity. At the same time, the single Cerebrolysin prescription, e.g., at the initially severe strokes, accompanied with both the focal and general cerebral symptoms, cerebral edema signs, is often not enough efficient, and requires its administration in combination with other anti-ischemic drugs. In addition, the strong dependence of the Cerebrolysin efficiency on the selected dose adequacy was revealed. The uncontrolled excessive Cerebrolysin dose augmentation often negatively affects the restorative processes rates and intensity. Hence, the main detriments of the known drug are the long term treatment; low activity and specificity due to the feeble neuroprotective effect; and extremely low regenerative and reparative potentials for the nervous tissue.

The Cerebrocurin contains the water-soluble prenatal neuropeptides and the products of cleavage of the inactive high molecular weight precursors of the animal embryos brain homogenate; this homogenate, diluted in physiological solution, is exposed to the immobilized proteolytic enzyme; the interaction conditions are defined on the basis of the target preparation check sample; and the resulting solution undergoes a 30-day cure at max. 10°C. This technique offers a higher (compared with the Cerebrolysin) activity of the resulting drug and significantly expands its spectrum of action, due to the higher mass of the peptides and low molecular weight proteins remaining after proteolysis. However, despite the long time solution formation before the aggregation and proteolysis processes end, this technique does not yield the purity required for the intravenous use; hence, the lesser bioavailability. The aqueous extraction without the solution, detergents, proteolysis inhibitors, and solubilizers buffering at the Cerebrocurin production phases, and the undetermined animal brain gestation terms does not allow for the necessary standardization; hence, the significant discrepancies as of the evolutionarily fixed protein-peptide concentrations ratios, and lower tissue-specific neuroregenerative activity and pharmacologic effect stability pharmacologic, all being limited to the feedback type biologic effect due to the tissue proteolysis products signal function.

The Cortexin contains the complex of the water-soluble biologically active alkaline, acid, and neutral polypeptides with the 500 to 15000 Da molecular mass and 3.5 to 9.5 isoelectric points. These are obtained from the livestock brain blenderized frozen tissue. The process consists in extracting with the zinc chloride containing acetic acid solution; separating the sediment; treating the supernatant liquid with acetone; drying with further and purifying, sterilizing, and freeze drying the target product. The drug is intended for the intramuscular and intravenous administration, the biologically active low molecular weight components concentration is low; hence, the treatment is high dose and long term. Though the Cortexin participates in regulating the inhibitory and exciting amino acids ratios, and concentrating the serotonin and dopamine, has a GABA-positive effect, reduces the toxic effects of the neurotropic drugs, improves the learning and memory processes, accelerates the brain functions rehabilitation after stresses, its activity and specificity are not high due to the feeble neuroprotective effect, its regenerative potential is low and reparative potential is insignificant for the nervous tissue; hence, the long term protracted treatment.

The Cerebrolysate also contains the water-soluble polypeptides obtained in the bovine brain cortex hydrolysis; their enzymatic activity is feeble; their molecular mass is max. 15000 Da. The mentioned in the Patent variation of the drug amino acid composition admits a significant compositional variation of the resulting peptide concentration ratios, determining the low tissue-specific activity and pharmacologic effect stability with the feeble neurometabolic and unexpressed nootropic effects.

Semax (Met-Glu-His-Phe-Pro-Gly-Pro) : synthetic analog of the ACTH/4-10/ fragment, where all the heptapeptide amino acids are L-forms. The drug has effect on the processes associated with the memory formation and learning. It improves the selective attention when learning and analyzing information; the memory trace consolidation; and the organism adaptation to the hypoxia, cerebral ischemia, general anesthesia, and other noci-influences. Under the neuropsychic fatigue, it facilitates the attention focusing, improves the operator performance, favors the mental capacity retention and accelerates its restoration, as prolonged action memory stimulator.

Today, on the background of the implacable vascular, toxic, infectious, and autoimmune genesis nervous system diseases incidence growth, no drugs of the nervous system specific direct reparants group are available.

**Disclosure of Invention**

The technical task of the claimed Invention is to improve the efficiency of the acute cerebrovascular accidents treatment method using the high efficiency drug, protein-polypeptide complex, conditions and dosages selection when treating the cerebrovascular and spinal circulation accidents, in function of the disease character and patient's status, and pathological process stage, to reduce the protracted treatment term; and to ensure the positive dynamics of the patients' objective neurologic indicants.

The problem solution consists in the acute cerebrovascular accidents treatment method by administering, subcutaneously, intramuscularly, intranasally, or intrathecally, from the first hours of the acute focal neurologic symptoms, the 0.05 to 8.0 mg/day pharmaceutical composition with the tissue-specific reparative effect on the nervous tissue, containing the protein-polypeptide complex and pharmaceutically acceptable diluent, containing the officinal buffer solution with the excipients containing the high molecular weight compounds, stabilizers, preservatives, and solubilizers, which protein-polypeptide complex is obtained from the mid first third to mid last third gestational age livestock embryonic brain tissue, which complex contains the negatively charged faintly acid neutral proteins and polypeptides relating to the growth factor, differentiation factor, signaling molecules, and intercellular adhesion molecules, that determine its biologic and pharmacologic activity, with the 5 to 200 kDa molecular masses, wherein at least 80% of the total protein mass has the 10 to 120 kDa molecular mass, and characterized by a peak at the 274-284 nm wave length in the UV-visible spectrum and bands in the pI 4.2 to 8.4 range at isoelectric focusing in a 5% polyacrylamide gel.

In function of the patient status severity, the said pharmacological composition is administered subcutaneously, intramuscularly, intravenously, intranasally, or intrathecally, from the first hours of the acute focal neurologic symptoms, wherein the claimed method exercise uses the following said pharmacological composition dosages and administration routes.

Moderate severity (Glasgow coma scale aggregate clinical score: min. 12; stroke scale (NIHSS) aggregate clinical score: max. 7) : recommended doses: 0.1 to 0.2 mg (1 to 2 mg 0.1% solution), subcutaneously or intramuscularly, 1 to 2/day; or intranasally 0.1% solution 200 to 400 μl into every nasal passage, morning and day time, 7 to 10 days, renewing the treatment session in the early rehabilitation period, in 5 to 10 days.

Grave condition (as of the date of admission: Glasgow coma scale aggregate clinical score: min. 7, max 12; stroke scale (NIHSS) aggregate clinical score: min 7, max. 14) : recommended doses: 0.2 to 0.6 mg daily dose (2 to 3 ml 0.1% solution), subcutaneously or intramuscularly, 1 to 2/day; or slow intravenous infusion, 0.2 to 0.4 mg (2 10 4 ml 0.1% solution) 1/day, 5 to 10 days, renewing the treatment session in the early rehabilitation period, in 5 to 10 days.

At the extreme patient condition as of the date of admission, with expressed impairment of consciousness, the drug (pharmaceutical composition containing protein-polypeptide complex) dosages are: 0.4 to 0.8 mg, slow intravenous infusion 0.2 to 0.4 mg (2 to 4 ml 0.1 % solution), 1 or 2/day, 7 to 10 days; or intrathecally, 0.2 to 0.4 mg (2 to 4 ml 0.1 % solution), once every 2 to 4 days (at no absolute counterindications to lumbar puncture: further on, intravenously or subcutaneously, relevant single or course doses, in function of the gravity).

## Best Embodiment of Invention

Hence, the embodiment of the acute cerebral and spinal circulation accidents treatment method uses the new neuroprotective drug that stimulates the nervous tissue physiologic and reparative regeneration; the drug is the protein-polypeptide complex obtained from the hoof livestock brain embryonic tissue, with the 5 to 200 kDa molecular masses of the component neutral and faintly acid proteins and polypeptides, that are growth factors, differentiation factors, and signaling molecules; and the dosage is 0.1 to 0.8 mg (1 to 4 ml 0.1% solution) 1 to 2/day subcutaneously, intramuscularly, or intravenously; or intranasally 0.1% solution, 0.2 to 0.4 ml into every nasal passage, morning and day time, 7 to 14 days, renewing the treatment session in 5 to 14 days. Recommended dosage at severe strokes: up to 0.6 mg/day; extreme severity with expressed impairment of consciousness: up to 0.8 mg/day; or intrathecally, 0.4 mg once every 2 to 4 days, at no absolute counterindications to lumbar puncture. The method is favorable as of the restorative processes rates and intensity and induces the accelerated regression of the focal and general cerebral lesions (see Tables 3 to 4).

Thus, the claimed method uses the pharmaceutical composition wherein the pharmacologic substance (biologically active substance) is the protein-polypeptide complex obtained as follows:

- the quick-frozen mid first third to mid last third gestational age hoof livestock embryonic brain is gradually unfrozen, and, step by step, within the temperature ranges 2°C to 28°C:

- is homogenized in buffer solution, simultaneously extracting in presence of the buffer solution proteolysis reversible inhibitors and nonionic detergents at pH min. 5.2 and max. 8.5, at the solution / tissue volume ratios max. 1:0.5;

- the homogenate is separated from the undissolved tissular and cellular components by centrifuging at 10,000 g to 30,000 g during 90 to 30 minutes, respectively;

- the supernatant is stepwise filtered down to the max. 0.45 $\mu$m mesh filters;

- the filtrate undergoes the anion-exchange chromatography using a saline buffer solution as mobile phase and separating the linked to sorbent proteins and polypeptides in stepwise gradient, with the help of a 0.08 to 0.26 mol/l ionic strength and 5.2 to 8.5 pH eluent, increasing the mobile phase ionic strength in the 0.02 mol/l steps, and beginning to collect the target fractions with the min. 0.1 ionic strength solution; and

- the collected target fractions are desalinized in dialysis or gel filtration, and, having added the bacteriostatic and fungicidal preservatives, 0.06 mg/ml max. total concentration, and solubilizer, 0.01 mg/ml max. total concentration, are ultra filtered at max. 0.22 $\mu$m mesh, and then sterile packed.

The resulting complex contains the 5 to 200 kDa molecular mass negatively charged faintly acid neutral proteins and polypeptides relating to the growth factor, differentiation factor, and signaling molecules, determining its biologic activity, wherein min. 80% of the total protein mass lie in the 10 to 120 kDa range, and wherein a peak exists in the 274 nm to 284 nm wave length range in the UV visible spectrum and bands exist in the pI 4.2 to 8.4 range at isoelectric focusing in a 5% polyacrylamide gel.

To keep with the order of description of the claimed technical solution, without unnecessary encumbrance, the more detailed information on the complex is given in the end part of this description.

The so obtained biologically active complex (protein-polypeptide complex) is the basis for production of the pharmaceutical composition containing the protein-polypeptide complex in the therapeutically efficient amounts; and pharmaceutically acceptable excipients, including the pharmaceutically acceptable diluent in form of officinal buffer solution (e.g., phosphate buffer), high molecular weight substances (e.g., carmellose), stabilizers (e.g., mannitol), preservatives (e.g., Nipagin), and solubilizers (e.g., Polysorbate 80).

**Pharmaceutical composition production example.**

The obtained porcine embryonic brain biologically active complex solution is diluted with the 50 mM glycin-phosphate buffer solution, containing the preservatives (Nipagin or benzalkonium chloride) and solubilizers (mannitol, carmellose, Polysorbate 80) in officinally acceptable concentrations, and disubstituted sodium phosphate to pH 8.0 to get the final total protein in resulting solution concentration within the 0.9 to 1.2 mg/ml range.

The resulting pharmaceutical composition is sterilizing filtered, through a max. 0.1 $\mu$m mesh membrane filter, dispensed in glass vials, and plugged.

To keep with the order of description of the claimed technical solution, without unnecessary encumbrance, the more detailed information on the complex is given in the end part of this description.

Hence, the said protein-polypeptide complex component of the claimed pharmaceutical composition functions as an efficient medicinal drug in the acute, subacute, and early rehabilitation periods of the early rehabilitation periods of the cerebrovascular accidents, both ischemic and hemorrhagic, with the advice of the appropriate daily and course dosage schedules, in function of the pathologic process and patients' gravities. The biologically active protein-polypeptide complex biological and pharmacological activities are due to the efficient evolutionarily fixed tissue-specific concentration ratios of the water-soluble negatively charged faintly acid neutral proteins and polypeptides relating to the growth factor, differentiation factor, and signaling molecules, ensuring thus the tissue-specific reparative-regenerative effect. However, the possibility and peculiarities of this known complex application as remedy for the acute cerebrovascular accidents has not been described, yet.

The clinical efficiency of the protein-polypeptide complex application, with validation of the dosage schedules in function of the character, patient gravity, and pathological process stage, reduction of the protracted treatment term, and positive dynamics of the patients' objective neurologic indicants when treating the acute cerebrovascular accidents was confirmed by the multicenter randomized comparative open clinical study. Together with the protein-polypeptide complex safety, tolerability, and absence of any adverse side effects, the drug application from the first hours of the cerebrovascular accident resulted in the lower general cerebral and focal neurologic symptoms, up to the complete restoration of the lost functions. The complex showed the best activity as of the motor disturbances regression in the patients in the cerebral stroke acute period. In this case, to the 6th to 10th days, the motor sphere neurologic deficiency intensity reduced almost two fold compared with the clinical control having received a comparable comprehensive differentiated treatment. The focus size instrumental (CT and MRI) visualization showed a certain decrease of the cerebral tissue lesion area and perifocal edema compared with the clinical control.

The said study involved a total of 120 acute cerebrovascular accident patients, of which 60 were in the control group. All the patients received, from the moment admission to clinic, the comprehensive differentiated treatment, including the respiratory and central and cerebral hemodynamics disorders correction, hemorheology correction, and, when necessary, dyscrasia correction, with the laboratory indicants dynamic control. No patients were administered the metabolically active drugs during the whole stroke acute period. This allowed to assess, using the clinical scales (NIHSS, Information-Memory-Concentration Test, Speech Questionnaire, and Barthel Index), and dynamic laboratory instrumental examination (brain CT and MRI; cerebral vessels ultrasonic intracranial duplex scanning; and blood clinical, biochemical, and rheological tests) the effects of the protein-polypeptide complex under study on the nervous and vascular systems intercourse alteration pathophysiological and pathomorphological processes on the background of the comprehensive differentiated treatment widely used in various neurologic inpatient clinics. The examined patients group did not include the subjects with the peripheral nerve, intrinsic degenerative, autoimmune, and infectious diseases. The patients whose acute stroke diagnosis was not confirmed by the additional brain computer or magnetic resonance tomography results were excluded from the group.

**Clinical characteristic of acute cerebrovascular accident basis therapy patients randomized into control group:** for the control group distribution of the acute cerebrovascular accident patients by age, sex, lesion character and area see Table 1 in Annex.

60 acute cerebrovascular accident patients (50% examined: 32 male (53%) and 28 female (47%), ages 36 to 80) were randomized into the control group; they received the comprehensive differentiated treatment, including the euvolemic controlled hemodilution; fluid and electrolyte and acid-alkali balances to support the internal homeostasis; metabolic output clearance; and cerebrovascular system stabilization with arterial pressure correction.

Of the 60 baseline therapy patients, in 35 the cerebral ischemic lesions were verified in the following pathogenetic variants: 22 (37%) : lacunar infarction; 10 (16%) : atherothrombotic infarction; and 3 (5%) : embolic infarction.

In 25 (42%>) patients (13 male and 12 female) the hemorrhagic type acute cerebrovascular accident was verified; in 20 of them the parenchymatous hemorrhage in cerebral hemisphere was verified (left: 9, right: 11); in 5 patients the subarachnoid hemorrhage was revealed. At admission:- in 35 (58%) patients the status was assessed as moderately severe (NIHSS score: max. 18; Glasgow coma scale score: min. 12);

- in 18 (30%) the status was assessed as grave; and

- in 7 (12%) patients the status was assessed as extremely severe (NIHSS aggregate score: above 18; Glasgow coma scale score: below 12).

**Clinical characteristic of patients randomized into protein-polypeptide complex group**: 60 patients (50% examined: 36 male (60%) and 24 female (40%), ages 36 to 80) received, on the background of the comprehensive differentiated treatment, during 10 days and from the 21st to 28th days, the protein-polypeptide complex (ZAO Pharm-Sintez, Moscow), 1 to 4ml 0.1% solution, 1 to 2/day subcutaneously, intramuscularly, or intravenously; or intranasally 0.1% solution, 0.2 to 0.4 ml into every nasal passage, morning and day time, 7 to 14 days, renewing the treatment session in 5 to 14 days. Recommended dosage at severe strokes: up to 0.6 mg/day; extreme severity with expressed impairment of consciousness: up to 0.8 mg/day; or intrathecally, 0.4 mg once every 2 to 4 days, at no absolute counterindications to lumbar puncture.

Of the 60 drug under study patients, in 38 the cerebral ischemic lesions were verified in the following pathogenetic variants: 20 (33%) : lacunar infarction; 12 (20%) : atherothrombotic infarction; and 6 (10%) : embolic infarction. For the acute cerebral circulation ischemic accident patients distribution in function of the vascular region and patient gravity, see Table 2 in Annex. In 22 (37%) patients (12 male and 10 female) the hemorrhagic type acute cerebrovascular accident was verified; in 18 of them the parenchymatous hemorrhage in cerebral hemisphere was verified (left: 10, right: 8); in 4 patients the subarachnoid hemorrhage was revealed.

At admission:

- in 34 (57%) patients the status was assessed as moderately severe (NIHSS score: max. 18; Glasgow coma scale score: min. 12);

- in 17 (28%) the status was assessed as grave; and

- in 9 (15%) patients the status was assessed as extremely severe; of them four (1 with massive ischemic stroke and three with hemorrhagic stroke) received the drug intrathecally.

In 52 patients the signs of the cerebral and cardiac atherosclerosis were recorded; in 49 cases these were associated with the hypertensive disease. In 2 cases, in the patients younger than 50, the renal genesis (nephritis and urinary stone disease) arterial hypertension was observed; in 2 cases, the high arterial blood pressure was conditioned by the hypertensive disease malignant hereditary form. In 5 observations the diabetes mellitus was combined with the cerebral atherosclerosis and hypertensive disease. In 12 patients the myocardial infarction was present in the case history. The associated heart rhythm disorders had the constant shape auricular fibrillation form in 11 subjects and the paroxysmal atrial fibrillation form in 1 subject. In 47% cases the acute form strokes developed, with the maximal clinical symptoms within the 1st hour of disease. In 53% cases, the subacute stroke development was recorded (several hours). 72% patients could associate the disease emergence with a previous effect of an adverse factor. Such triggering factors were: physical overextension in 7 cases (in 5 of them combined with the abandon or irregular use of the antihypertensive drugs); psychoemotional overextension in 3 cases; alcohol abuse before stroke in 5 cases; and visit to sauna in 2 cases.

In most patients (78%) the stroke evolved on the background of the arterial hypertension, by 20 mm Hg and more above the "working" values. In 2 cases the disease debuted within the framework of the cardiocerebral syndrome on the background of the transient cardiac insufficiency; the arterial pressure dropped by 40 to 50 mmHg. In the other observations the arterial pressure remained within the limits of individual norm.

In 85% cases, the first symptoms of disease were the focal neurologic ones. In 21 observations (12 sinistrocerebral and 9 dextrocerebral strokes) the focal symptoms were associated with the general cerebral ones, such as general torpidity, dullness, non systemic vertigo, and/or non localized headache of various intensity. All the patients (60 subjects, 100%) came to the clinic within the first 20 hours from the moment of disease development; of them 18 (30%) : within the 4 to 6 hours; 25 (42%) : within 6 to 12 hours; and the remaining 17 (28%) within 12 to 20 hours.

Among the grave patients, 18 (30%) had the min. 18 NIHSS score and max. 12 Glasgow score. These were in line with the moderate disturbances of consciousness, down to the clouding level; the presence of the local meningeal symptoms; the alterations of the oculocephalic reflexes with the "doll's eyes"; and grave focal defect. In 8 (13%) patients the total clinical score was above 24 NIHSS and below 8 Glasgow. This meant the extreme gravity of the status: the deep disturbances of consciousness (down to sopor or coma); the signs of cerebral edema and secondary syndrome; the central respiratory disturbances of the rhythmic polypnea or Cheyne-Stokes type (in terminal stages: group, periodic, apneic respiration, and, finally, gasping); the various alterations of the oculocephalic reflexes; the expressed vegetative-trophic changes; and the grave focal disturbances of neurologic functions. Four extremely grave patients were administered the drug under study intrathecally (endolumbarly) on the 3rd and 6th days from the disease outset. In 3 patients (1 male and 2 female) the hemispheral stroke was verified (2 female with the parenchymatous hemorrhage in the right brain and 1 man with the massive hemorrhagic stroke in the left inner carotid system). In 1 female patient the massive subarachnoidal hemorrhage was verified.

**Results of protein-polypeptide complex clinical study in the patients with the acute cerebrovascular accident on the background of the comprehensive differentiated therapy**.

To assess the efficiency (objective clinical scores) and safety (tolerability indexes) of the protein-polypeptide complex, on the background of the comprehensive differentiated therapy, in the patients with the acute cerebrovascular accident, the multicenter randomized comparative open clinical study used the following clinical scales: Glasgow Coma Scale; Allen's Prognostic Score; NIHSS; Information-Memory-Concentration Test; and Speech Questionnaire. The dynamic laboratory instrumental exams of the patients included the cerebral computer or magnetic resonance imaging; cerebrovascular transcranial Doppler sonography with embolodetection; echocardiography to verify the cardiac cavities thrombogenesis; and blood parameters biochemical and clinical monitoring with main rheological indexes.

The study results analysis proved that the ten-day treatment with the protein-polypeptide complex, administered subcutaneously, intramuscularly, or intrathecally, in different gravity patients, of different cerebrovascular lesions characters and/or localizations, yields no grave adverse effects. Similar results were obtained at the retreatment in the early rehabilitation period, 21st to 28th days.

Table 3 in Annex gives the dynamics of clinical score in all the patients' groups treated with the complex on the background of the comprehensive differentiated therapy, in the stroke acute period. It visually shows the neurologic deficiency regression dynamics, more expressed in the group treated with the complex (especially to the third and sixth days from the disease outset) compared with the control group. Table 4 shows in more detail the analysis of the regression of both the general cerebral and focal neurologic symptoms and the higher cortical functions by comparison groups. The motor disturbances regression dynamics in the protein-polypeptide complex patients to the 6th and 10th days is almost two fold better than for the control group, continuing the paresis intensity reduction to the enc of the stroke acute period.

The Speech Questionnaire also shows that the speech disorders regression in the complex group is much better than in the control one; the Information-Memory-Concentration Test independently confirms this. The assessment of the "comprehension" parameter evolution in the above test did not reveal any significant advantages for the complex group compared with the control one.

Hence, the scores dynamics comparison data obtained using the international clinical scales for the comparison groups make possible the conclusion that the protein-polypeptide complex is efficient enough in the ischemic stroke acute period. The complex administration allows to significantly accelerate the regression of the motor and speech disorders in any patients of different gravity, as for both the character and localization of the cerebrovascular lesions.

A significant indicator of the protein-polypeptide complex efficiency at acute cerebrovascular accident is its capacity to influence the volume and regression of the lesion focus, with reduction of the perifocal edema intensity according to the computer or magnetic resonance imaging dynamic data. Though all the cerebral stroke clinical aspects are greatly influenced by both the lesion volume and localization, the drug's effect on these parameters can be indirectly assessed when selecting the homogeneous clinical groups with sufficient patient sampling. Table 5 shows the computer and magnetic resonance imaging results by comparison groups. Though the focus size reduction data (by almost 30%) at cerebral stroke repeat the experimental ones (obtained in the laboratory animals for the protein-polypeptide complex effect on the focal ischemic cerebral lesion by photo-induced thrombosis) they require a confirmation with more precise instrumental methods and larger clinical observations sampling.

Hence, the scores dynamics comparison data obtained using the international clinical scales for the comparison groups make possible the conclusion that the protein-polypeptide complex is efficient enough in the ischemic stroke acute period. The complex administration allows to significantly accelerate the regression of the motor and speech disorders in any patients of different gravity, as for both the character and localization of the cerebrovascular lesions.

In the extreme gravity (4 patients) group where the drug under study was administered intrathecally (endolumbarly) an insignificant positive dynamics was recorded, in form of the consciousness disturbances shift toward the deep sopor- coma (1 subject); however, in all the patients the expressed local meningeal symptoms remained, indicating the expressed perifocal edema. In 2 patients, the alterations of the oculocephalic reflexes with the "doll's eyes" were recorded. After the new endolumbar administration of the protein-polypeptide complex, to the 10th day, 3 patients showed, despite the impairment of consciousness, an unexpressed motor excitement in response to a feeble pain stimulation, a more active defense reaction, lesser expressed meningeal symptoms, and no oculocephalic reflexes. To the 15th day, the consciousness level of these patients was assessed as deep sopor. A good regression of the meningeal symptoms was recorded; the patients responded to the expressed pain and sound stimulation with the eyelids and healthy extremities movements; however, the gross focal neurologic symptoms with vegetative-trophic alterations remained. To the 21st day, the consciousness level of these patients corresponded to the sopor. To the 28th day, the patients responded to different stimulations by opening their eyes; in 2 patients the swallowing reflex appeared; attempts to perform simple tasks were recorded. In 1 patient, despite the insignificant motor excitement remaining one day after every endolumbar administration of the drug, the expressed meningeal symptoms and gross neurologic deficiency remained; periodical central respiration disorders (rhythmic polypnea type), and various alterations of the oculocephalic reflexes with expressed vegetative-trophic disturbances were recorded. To the end of the study, the insignificant regression of the general cerebral and meningeal symptoms was recorded, without positive dynamics in the neurologic status to the 28th day.

The protein-polypeptide complex group fatality analysis revealed that 67% deaths occurred in the initially extremely grave patients (total clinical scores: max. 8 Glasgow scale, and below 24 NIHSS). The lethality rate in the protein-polypeptide complex group was 5% (3 patients), which is certainly lower than the 12% (7 patients) in the control group; with the commeasurably greater number of the initially grave patients in the protein-polypeptide complex group (see Tables 1 and 2).

Deaths: 1 grave patient, age 78, ischemic stroke in brain stem; and 1 extremely grave patient, age 73, massive hemorrhagic stroke in the right hemisphere (progressive evolution with death in the stroke most acute period); on the 5th and 3rd days, respectively. In the both cases the death occurred under the growing cerebral edema and developing dislocation syndrome with disorder of vital functions. In these patients, the expressed concomitant chronic somatic pathology in decompensation stage with acute cardiovascular disorders was recorded. In the third case, the sudden death on the 5th day occurred in the age 53, medium severity patient, with dextrocerebral hemorrhagic stroke, good regression of the general cerebral and focal neurologic symptoms (NIHSS 9 initially at admission to 5 to the third day, due to the positive dynamics of the motor disturbances). The supposed cause was, taking into account the clinic pattern, the pulmonary artery thrombembolia. Regretfully for the attending physician and primary investigator, the testament of the patient and wish of the relatives prevented from the pathomorphological exam. The absence of the clinically significant alterations in the clinical and biochemical blood and urine analyses and in the coagulogram and ECG should be noted.

Among the control group deaths, the massive ischemic hemisphere lesion patients and the concomitant chronic somatic pathology in decompensation stage or acute cardiovascular disorders ones prevailed. In 2 cases the cause of death was the pulmonary artery thrombembolia. In the other cases the deaths occurred under the growing cerebral edema and developing dislocation syndrome with disorder of vital functions.

When analyzing the blood laboratory clinical, biochemical, and rheological results, the higher fibrinogen and SFC levels in practically all the patients attract attention. This growth was more expressed in the hemorrhagic stroke patients; in the complex patients group, it was doubtful. The literature analysis allows a direct correlation between the brain lesion volume, fibrinogen (and, hence, SFC) level growth, and the inflammatory reaction intensity. Some authors associate the fibrinogen level growth with the reparative processes activity, reflected in the protein-polypeptide complex group.

Hence, the multicenter randomized comparative open clinical study proved the safety and clinical efficiency of the protein-polypeptide complex drug in treating the acute cerebrovascular accident patients. The clinically significative effect of the protein-polypeptide complex on the survivability, dynamics and terms of the lost functions restoration in the stroke acute period patients was shown.

See below the method embodiment examples, which illustrate it but do not limit the scope of claims.

**Example 1**. Patient M., male, age 68, diagnosis at admission: acute cerebrovascular accident, mixed type, left internal carotid artery, 6 hours after disease outset. No complaints expressed due to speech disorders. Past case history: ischemic heart disease, auricular fibrillation, hypertensive disease stage III. The acute cerebrovascular accident was of acute character, in form of sudden speech disorder and weakness of right extremities on the background of the auricular fibrillation paroxysm and arterial pressure growth. No loss of consciousness. Objective: no general cerebral and meningeal symptoms at admission. Sensorimotor aphasia, limited understanding of events. Central paresis of 7, 12 pairs of cerebral nerves on the right; right sided spastic hemiparesis, up to 3 in distal portions of hand, up to 4 in leg; anisoreflexia d>s; hemihypesthesia on the right. Total clinical scores: Glasgow coma scale: 9; NIHSS: 10; Speech Questionnaire: 9; and Information-Memory-Concentration Test: 21. The perfusion weighted MRI revealed, in the frontoparietal lobe on the left, a cortico-subcortical ischemic focus, with up to 9 $cm^3$ secondary diapedetic infiltration and perifocal area with up to 12 $cm^3$ edema. Treatment: on the background of the unified basic therapy: 0.1% protein-polypeptide complex solution, 0.1 mg subcutaneously, 2/day, during 10 days; then intranasally 400 $\mu$l (200 $\mu$l into every nasal passage, morning) 21st through 28th days. Result: on the background of the drug first administration: lesser disorientation; lesser auditory speech disorders to the end of the second day. On the third day of treatment: lesser motor speech disorders; "poor", but phrasal speech; sufficient understanding of events; and regression of the hand and leg paresis to 4. To the acute period end, on the 28th day, the patient was discharged from hospital due to the complete restoration of the speech function and complete regression of the motor sensitive disorders. In the neurologic status, as of the moment of discharge: anisoreflexia d>s without pathological foot signs. Clinical control: to 10th day: Glasgow coma scale: 11, NIHSS: 7, Speech Questionnaire: 10, and Information-Memory-Concentration Test: 26; to the 21st day: Glasgow coma scale: 12, NIHSS: 6, Speech Questionnaire: 15, and Information-Memory-Concentration Test: 29; and to the 28th day: Glasgow coma scale: 15, NIHSS: 4, Speech Questionnaire: 18, and Information-Memory-Concentration Test: 32. The new MRI on the 21st day showed the smaller, 8 $cm^3$, mixed stroke focus; and up to 9 $cm^3$ edema perifocal

zone. To the 28th day: reduced to 5.5 cm$^3$ mixed stroke focus without perifocal edema.

**Example 2**. Patient K., female, age 38. Diagnosis at admission: acute cerebrovascular accident, hemorrhagic type, right brain hemisphere. Admitted 4 hours after disease outset. No complaints expressed due to severe state. Past case history: aneurism of right medial cerebral artery. Disease outset: at physical exertion; then gradually growing weakness of left extremities, alteration of speech, and depression of consciousness. Objective: at admission, impairment of consciousness to sopor; moderate meningeal syndrome; central paresis of 7, 12 pairs of cerebral nerves on the right; left sided hemiplegia; anisoreflexia d>s; and hemianesthesia on the right. The computed tomography revealed a massive, up to 24 cm$^3$, hemorrhagic focus in the brain right hemisphere, with ventricular system deformation. Total clinical scores: Glasgow coma scale: 7; NIHSS: 18; Speech Questionnaire: 4; and Information-Memory-Concentration Test: 5. Treatment: on the background of the unified baseline therapy, 0.1% protein-polypeptide complex solution, 0.2 mg intrathecally, on the first and third days; from the fourth day, 0.2 mg intravenous infusions on physiological solution, once daily, seven days; then, subcutaneously, 0.4 mg once daily, 21st through 28th days. Result: on the background of the drug first administration, the patient showed the acceleration of the directed response to the external stimuli in the healthy extremities; after the second administration, the "evocation" accelerated ion response to the sound and nociceptive stimuli. To the end of the third day, the disorders of consciousness fully regressed; the place and self personality understanding signs appeared, together with the elementary contacts capacity: understanding of simple questions and tasks; the own speech on the "Yes"- "No" level appeared; minimal motions in the lower extremity became possible. To the fifth day, the meningeal syndrome regressed significantly; the perceptible speech range expanded; and the lower extremity paresis reduced to 2.5. To the end of the stroke acute period (21st day) the patient showed a significantly improved articulation, with capacity to bend the left leg at the knee; however, the upper extremity motions did not restore; the sensitivity disorders reduced. It should be noted that, despite the massive brain lesion focus and gross motor defect, the general cerebral and meningeal symptoms quickly regressed; the disease evolution was regredient; no trophic disorders developed. Clinical control: to the 10th day: Glasgow coma scale: 8, NIHSS: 16, Speech Questionnaire: 9, and Information-Memory-Concentration Test: 11; to the 21st day: Glasgow coma scale: 10, NIHSS: 14, Speech Questionnaire: 12, and Information-Memory-Concentration Test: 15; and to the 28th day: Glasgow coma scale: 12, NIHSS: 12, Speech Questionnaire: 15, and Information-Memory-Concentration Test: 21. The new MRI on the 21 st day showed the reduced to 14 cm$^3$ mixed stroke focus; and up to 8 cm$^3$ edema perifocal zone.

**Example 3**. Patient L., female, age 69. Diagnosis at admission: acute cerebrovascular accident, ischemic type, right internal carotid artery system. Hospitalized 3 hours after disease outset; complaints: weakness in left extremities. Past case history: hypertensive disease stage II. The disease outset was acute; the patient fell down (without losing consciousness) on the background of a stress situation, because of a sudden weakness in the left extremities. Objective: the consciousness is clear; however the patient is aspontaneous, misjudges her defect, and is not critical to the disease; moderate meningeal syndrome; look paresis to the left; central paresis of 7, 12 pairs of cerebral nerves on the left; cortical dysarthria; choking at swallowing because of the repressed pharyngeal reflex on the left; and left side deep spastic hemiparesis, up to 2 in the distal and 3 in the proximal portions of the extremities. Anisoreflexia s>d. Failure of complex sensitivities on the left; central disorders of urination. The computed tomography revealed a massive, up to 18 cm$^3$, hemorrhagic focus in the brain right hemisphere, and expressed perifocal  edema, up to 12 cm$^3$, with ventricular system deformation. Total clinical scores: Glasgow coma scale: 8; NIHSS: 16; Speech Questionnaire: 5; and Information-Memory-Concentration Test: 7. Treatment: on the background of the unified basic therapy: 0.1% protein-polypeptide complex solution, 0.2 mg subcutaneously, 2/day, during 10 days; then intranasally 400 $\mu$l (200 $\mu$l into every nasal passage, morning and day time) 21st through 28th days. Result: after the first administration of the drug, the patient began to participate more actively in conversation, became more critical as for her status; to the end of the first day and on the second day she objectively evaluated her defect; the meningeal symptoms reduced; and the hand and foot motions grew to 3. To the fifth day from the disease outset, the meningeal syndrome completely regressed; the range of the eye globes motions restored; the swallowing normalized. On the 15th day, the hemiparesis reduced to 4; the urination disorders disappeared. To the 21st day, the sensitivity disorders regressed; the speech was articulate; only minimal muscular weakness remained in the left extremities; the muscular tone in the lower extremity normalized; the s>d anisoreflexia remained; and the flexion and extension type pathological foot signs remained. No trophism disorders were recorded during the whole stroke. Clinical control: to the 10th day: Glasgow coma scale: 10, NIHSS: 15, Speech Questionnaire: 14, and Information-Memory-Concentration Test: 17; to the 21st day: Glasgow coma scale: 12, NIHSS: 14, Speech Questionnaire: 17, and Information-Memory-Concentration Test: 18; and to the 28th day: Glasgow coma scale: 12, NIHSS: 10, Speech Questionnaire: 19, and Information-Memory-Concentration Test: 25. The new MRI on the 21st day showed the reduced to 12 cm$^3$ mixed stroke focus, without perifocal zone and ventricular system compression.

**Example 4**. Patient M., male, age 56, diagnosis at admission: acute cerebrovascular accident, ischemic type, Desproges-Gotteron artery system, 6 hours after disease outset. Complaints: aching pain in lumbar region, weakness in lower extremities with impaired sensitivity and range of motions. Past case history: hypertensive disease stage II; lumbar spine osteochondrosis; compression fracture of 2nd lumbar vertebra (traffic accident); and hernias of the L2-L3, L4-L5-S1 intervertebral disks. The disease developed acutely: after a fall down (on glaze ice) the sudden sensation disorders appeared from the waist down, with weakness in legs, on the background of the arterial pressure growth. No loss of consciousness. Objective: no general cerebral and meningeal symptoms at admission. Cerebral nerves: no peculiarities; soft lower paraplegia, up to 4 in the proximal and 2.5 in the distal portions; bilateral hyporeflexia; and parahypesthesia of the superficial sensitivity from L2 D• S level and of the deep sensitivity from L5 level. Total clinical scores: Glasgow coma scale: 12; NIHSS: 10. The perfusion weighted MRI revealed, in the D12- L1 spinal cord segments the lowered density zone, of the ischemic focus type, 0.8 to 0.9 $cm^3$, with unexpressed up to 0.5 $cm^3$ perifocal edema. Treatment: on the background of the unified basic therapy: 0.1% protein-polypeptide complex solution, 0.2 mg subcutaneously, 2/day, during 10 days; renewing the treatment session in 5 days. Result: to the end of the second day of administration the pain syndrome reduced. On the fifth day, the muscle tone growth in the proximal portions was recorded; the range of motions grew up to 4. To the acute period end, the patient was dismissed from hospital, with the complete restoration of motion in the lower extremities proximal portions. The distal portions motion range grew up to 4. The deep sensitivity disorders regressed completely. In the neurologic status, as of the moment of discharge: lowered knee and foot reflexes with feeble anisoreflexia d>s without pathological foot signs. Clinical control: to the 10th day: Glasgow coma scale: 12, NIHSS: 7; to the 21st day: Glasgow coma scale: 13, NIHSS: 6; and to the 28th day: Glasgow coma scale: 15, and NIHSS: 4. The new MRI on the 21st day showed the reduced to 8 $cm^3$ mixed stroke focus; and up to 9 $cm^3$ edema perifocal zone. To the 28th day: reduced to 0.5 $cm^3$ mixed stroke focus; no perifocal edema.

**Example 5**. The dependence of the various gravities patients' treatments efficiency on the protein-polypeptide complex dosage was studied. The following clinical scales were used: Glasgow coma scale, NIHSS, Speech Questionnaire, and Information-Memory-Concentration Test. These showed the total clinical score growth to the 6th, 10th, 21st, and 28th days of stroke, respectively. The groups of patients are noted having received 0.1; 0.2; 0.4; and 0.8 mg/day of the protein-polypeptide complex, subcutaneously, intravenously, intrathecally, and intravenously. The obtained data show the high efficiency of the dosages:

- medium severity ischemic stroke patients: 0.2 mg/day subcutaneously in acute period; then intranasally, 400 μl/day 0.1% solution (200 μl into every nasal passage, morning and day), early rehabilitation period (21st through 28th days);

- grave patients: 0.4 mg intravenously, then intranasally, 400 μl 0.1% solution (200 μl into every nasal passage, morning and day time), early rehabilitation period; and

- extreme ischemic stroke patients: intrathecally, on the first and third days, 0.4 mg, then 0.8 mg subcutaneously, up to the 10th day, then 0.2 mg subcutaneously once a day, early rehabilitation period.

Thus, the comparison of the protein-polypeptide complex dosages and administrations efficiencies revealed, for the medium gravity group, the somehow higher efficiency of the 0.2 mg/day (2 μg/kg in average) dose compared with the higher ones. However, in the grave patients group, the clinical dynamics was somehow more expressed at the 0.8 mg/day (10 μg/kg in average) dosage.

Tables 1 to 5 below graphically demonstrate the efficiency of the claimed method of treatment of the acute cerebrovascular accidents.

**Table 1. Patients distribution by age, sex, lesion character and area, control group**

**60 acute cerebrovascular accidents patients (32 (53%) male and 28 (47%) female)**

| 35 ischemic stroke patients | | | 25 hemorrhagic stroke patients | | |
|---|---|---|---|---|---|
| (19 male and 16 female) | | | (13 male and 12 female) | | |
| **Lacunar** | **Atherothrom botic** | **Embolic** | **Left hemisphere** | **Right hemisphere** | **Subarachnoid al hemorrhage** |
| 22 | 10 | 3 | 9 | 11 | 5 |
| **Left mesencephalic artery** | **Right mesencephalic artery** | **Vert.- basill.** | | | |
| 12 | 11 | 12 | | | |

| **Medium gravity** | | **Grave** | | **Extreme gravity** | | **Medium gravity** | | **Grave** | | **Extreme gravity** | |
|---|---|---|---|---|---|---|---|---|---|---|---|
| 19 | | 12 | | 4 | | 16 | | 6 | | 3 | |
| Male | Female | Male | Female | Male | Female | Male | Female | Male | Female | Male | Female |
| 11 | 8 | 8 | 4 | 1 | 3 | 7 | 9 | 4 | 2 | 2 | 1 |

EP 2 656 853 A1

**Table 2. Distribution of protein-polypeptide complex patients by age, lesion localization, and status gravity.**

**60 acute cerebrovascular accidents patients (32 (53%) male and 28 (47%) female)**

| 35 ischemic stroke patients (24 male and 14 female) | | | 25 hemorrhagic stroke patients (12 male and 10 female) | | |
|---|---|---|---|---|---|
| Lacunar | Atherothrombotic | Embolic | Left hemisphere | Right hemisphere | Subarachnoidal hemorrhage |
| 20 | 12 | 6 | | | |
| Left mesencephalic artery | Right mesencephalic artery | Vert.- basill. | 10 | 8 | 4 |
| 16 | 12 | 10 | | | |

| Medium gravity | | Grave | | Extreme gravity | | Medium gravity | | Grave | | Extreme gravity | |
|---|---|---|---|---|---|---|---|---|---|---|---|
| 21 | | 12 | | 5 | | 12 | | 6 | | 4 | |
| Male | Female | Male | Female | Male | Female | Male | Female | Male | Female | Male | Female |
| 14 | 7 | 8 | 4 | 2 | 3 | 6 | 4 | 4 | 3 | 2 | 3 |

# EP 2 656 853 A1

**Table 3. Dynamics of NIHSS in groups of patients studied.**

| Study time | | | | | | | | | | | | | |
|---|---|---|---|---|---|---|---|---|---|---|---|---|---|
| 1st day | | 3rd day | | 6th day | | 10th day | | 15th day | | 21st day | | 28th day | |
| • | ± m | • | ± m | • | ± m | • | ± m | • | ± m | • | ± m | • | ± m |
| Protein-polypeptide complex | | | | | | | | | | | | | |
| 11 | 3 | 8.2 | 2.5 | 6 | 2.3 | 4.4 | 2.2 | 4.4 | 2.1 | 3.6 | 2.1 | 3.5 | 2.4 |
| Control group | | | | | | | | | | | | | |
| 10.7 | 3.4 | 10.2 | 3.2 | 9 | 2.7 | 7.8 | 2.9 | 8.1 | 3.3 | 7.2 | 2.8 | 6.4 | 2.7 |

**Table 4. Dynamics of general cerebral and focal neurologic symptoms regression, and higher cortical function restoration by comparison groups.**

| Study time | | | | | | | | | | | | | |
|---|---|---|---|---|---|---|---|---|---|---|---|---|---|
| 1st day | | 3rd day | | 6th day | | 10th day | | 15th day | | 21st day | | 28th day | |
| • | ± m | • | ± m | • | ± m | • | ± m | • | ± m | • | ± m | • | ± m |
| Dynamics of motor disturbances, NIHSS | | | | | | | | | | | | | |
| Protein-polypeptide complex | | | | | | | | | | | | | |
| 6.6 | 3 | 4.8 | 1.9 | 3.6 | 1.6 | 2.3 | 1.1 | 2.3 | 1.2 | 1.9 | 0.9 | 1.6 | 0.9 |
| Control group | | | | | | | | | | | | | |
| 6.3 | 3.2 | 5.9 | 2.8 | 5.1 | 2.8 | 4.7 | 2.7 | 4.8 | 3 | 3.7 | 2.8 | 3.3 | 2.5 |
| Dynamics of speech disorders regression, Speech Questionnaire | | | | | | | | | | | | | |
| Protein-polypeptide complex | | | | | | | | | | | | | |
| 11 | 5.8 | 13.8 | 4.3 | 15.4 | 2.7 | 18 | 2.3 | 17.1 | 2.1 | 17 | 2.4 | 18.1 | 1.9 |
| Control group | | | | | | | | | | | | | |
| 11.2 | 5.5 | 11.5 | 6.4 | 12.1 | 4.9 | 14.3 | 4.2 | 14.4 | 5.1 | 14.6 | 4.7 | 15.5 | 3.4 |
| Dynamics of "comprehension" parameter regression, Information-Memory-Concentration Test | | | | | | | | | | | | | |
| Protein-polypeptide complex | | | | | | | | | | | | | |
| 8• | 2.5 | 10.1 | 3.8 | 11.3 | 1.8 | 13.4 | 1.2 | 13 | 1.3 | 13 | 2.1 | 13.6 | 1.4 |
| Control group | | | | | | | | | | | | | |
| 8.5 | 3.3 | 8.9 | 3.5 | 9.2 | 2.8 | 9.3 | 2.7 | 9.4 | 2.9 | 9.6 | 2.8 | 10.3 | 2.2 |
| Dynamics of "comprehension" parameter regression, Information-Memory-Concentration Test | | | | | | | | | | | | | |
| Protein-polypeptide complex | | | | | | | | | | | | | |
| 4 | 1.4 | 3.8 | 1.9 | 4.1 | 1.8 | 4.6 | 1.2 | 4.6 | 1.3 | 4 | 1.5 | 4.5 | 1.2 |
| Control group | | | | | | | | | | | | | |
| 4.1 | 2 | 4 | 2.2 | 4.1 | 2.3 | 4.4 | 2.2 | 4.3 | 1.9 | 3.9 | 1.8 | 4.4 | 1.9 |

**Table 5. Dynamics of aggregate brain focal lesion in comparison groups, computed and magnetic resonance imaging.**

| Comparison groups | Lesion focus size (cm) | | | |
|---|---|---|---|---|
| | At admission (1st day) | | 21st day | |
| | • | ± m | • | ± m |
| Protein-polypeptide complex | 1.82 | 1.2 | 1.2 | 1.1 |
| Control group | 1.76 | 1.18 | 1.51 | 1.2 |

As promised at the beginning of this description, the Applicant gives below the more detailed information concerning the protein-polypeptide complex contained as pharmacologic substance (biologically active substance) in the pharmaceutical composition used in the claimed Invention.

The desired objective is reached by that the pharmaceutical composition intended to treat the vascular, traumatic, toxic, hypoxic, and autoimmune genesis central and peripheral nervous system diseases has the form of the solution for parenteral, intranasal, and subconjunctival administration; and its active ingredient is the 0.01 to 2.0 mg/ml biologically active protein-polypeptide complex with the tissue-specific reparative effect on the nervous tissue, obtained from the mid first third to mid last third gestational age livestock embryonic brain tissue, which complex contains the negatively charged faintly acid neutral proteins and polypeptides relating to the growth factor, differentiation factor, signaling molecules, that determine its biologic and pharmacologic activity, with the 5 to 200 kDa molecular masses, wherein at least 80% of the total protein mass has the 10 to 120 kDa molecular mass, and characterized by a peak at the 274-284 nm wave length in the UV-visible spectrum and bands in the pI 4.2 to 8.4 range at isoelectric focusing in a 5% polyacrylamide gel, and which complex contains a pharmaceutically acceptable diluent.

With all this, the preferable pharmaceutical composition diluent is an officinal buffer solution with excipients, including the high molecular weight compounds, stabilizers, preservatives, and solubilizers.

Thus, the described pharmaceutical composition contains the active ingredient, which is the biologically active protein-polypeptide complex consisting of the 5 to 200 kDa molecular mass negatively charged faintly acid neutral proteins and polypeptides, obtained from the mid first third to mid last third gestational age livestock embryonic brain tissue, which complex contains the organo-specific nervous tissue growth and differentiation factors, and signaling molecules, in therapeutically efficient ratios and concentrations; and it contains the pharmaceutically acceptable diluent, consisting of the buffer solution, e.g., phosphate buffer, and excipients: high molecular weight compounds, e.g., carmellose; and it contains the stabilizers, e.g., mannitol, preservatives, e.g., Nipagin, and solubilizers, e.g., Polysorbate 80.

The claimed pharmacologic composition, which consists of the protein-polypeptide complex and pharmaceutically acceptable diluent differs from the prior substances and compositions extracted from the animal brain, as of the feedstock source at the fixed gestational ages, as of the molecular masses (max. 200 kDa) and qualitative composition of the protein-polypeptide fractions, as of the evolutionarily fixed in ontogenesis growth proteins, differentiation factors, and signaling molecules concentration ratios, as of the method of administration (parenteral, intrathecal, intranasal, subconjunctival), as of the safety of use (no toxicity, mutagenicity, carcinogenicity, and/or allergenicity), and as of the biological and pharmacological activity and this activity specificity (tissue- and organo-specific, reparative-regenerative).

The examples below illustrate the claimed technical solution.

**Example 6. Biologically active complex production method.**

Protein fraction production from porcine embryonic brain. 250 grams of quick-frozen porcine embryonic brain, 14 to 18 weeks gestational age, is unfrozen and 7-minute homogenized in 1200 ml of 0.05 M TRIS-glycin-phosphate buffer, pH 7.0, containing 1 mM EDTA and 0.1% maltose, at 8°• . The homogenate is filtered through a dense cloth to separate the ballast substances, then it is centrifuged at 18, 000 g, and then filtered through a 0.45 $\mu$m mesh membrane filter at 8°• . The filtrate is then applied to a 200 ml chromatographic column with the Toyopearl DEAE-650M anion-exchange medium; the column is equilibrated with 4 volumes of the TRIS-glycin-phosphate buffer, pH 7.0, containing 0.1 mM EDTA and 0.1 mM NaCl. The proteins linked to the carrier are separated by the stepwise gradient of the 105-step increasing the salt concentration, and collecting the target fractions. Chromatography temperature: 8°C. The target fraction is stage by stage ultra filtered at max. 8.0 kfg/cm$^2$ back pressure, through 5 kDa and 200 kDa retention potential materials; then it is desalinized, and diluted with the 0.05 M glycin-NaOH solution, pH 7.4, down to the 1.0 mg/ml protein concentration. The solution is sterilizing filtered, through a max. 0.22 $\mu$m mesh membrane filter. To characterize the resulting protein fraction the ultraviolet spectrophotometry, gel chromatography, electrophoresis in polyacrylamide gel, and amino acid analysis were used. The solution ultraviolet spectrum is read in the 250 to 350 nm wave length range, the maximum absorption is observed at $280\pm5$ nm. To determine the molecular mass of the preparation forming proteins the following methods are used: gel chromatography with Superdex 75 (GE Healthcare) and denaturing SDS-Page electrophoresis in 12% polyacrylamide gel in comparison with standard marker proteins set. The column is calibrated with the protein standards, molecular masses range: 13 kDa to 200 kDa. The above methods have found that the drug contains the negatively charged faintly acid neutral proteins and polypeptides relating to the growth factor, differentiation factor, 5 to 200 kDa molecular mass signaling molecules; wherein at least 82% of the total protein mass have the 10 to 120 kDa molecular mass; and wherein a peak exists at the 274 nm wave length in the UV visible spectrum and bands exist in the pI 4.2 to 8.4 range at isoelectric focusing in 5% polyacrylamide gel; and which drug contains a pharmaceutically acceptable diluent.

Amino acid analysis (%) : Asp: 10.82 + 2.8; Thr: 5.4 + 1.6; Ser: 5.2 + 1.8; Glu: 16.2 + 3.4; Pro: 7.045 + 2.5; Gly: 5.2 + 2.4; Ala: 5.4 + 1.8; Val: 7.08 + 2.9; Met: 2.65 + 1.4; He: 4.45 + 1.8; Leu: 9.4 + 2.6; Tyr: 4.02 + 1.2; Phe: 4.8 + 1.9; Orn: 0.48 + 0.2; Lys: 8.48 + 2.4; His: 2.8 + 0.9; and Arg: 6.52 + 2.2.

**Example 7. Biologically active complex production method.**

Protein fraction production from the piglet porcine embryonic brain. 200 grams of quick-frozen piglet porcine embryonic brain, 3 to 4 weeks gestational age, is unfrozen and 5-minute homogenized in 800 ml of 50 mM TRIS-glycin-phosphate buffer, pH 5.8, containing 1 mM of ethylenediaminotetraacetic acid (EDTA) and 0.1% mannitol, at 25°• . The homogenate is filtered through a dense cloth to separate the ballast substances, then it is 30-minute centrifuged at 30, 000 g, and then filtered through a 0.45 $\mu$m mesh membrane filter at 25°• . The filtrate is then applied to a 250 ml volume chromatographic column with the DEAE-Sepharose anion-exchange medium; the column is equilibrated with 4 volumes of the maleate buffer solution, pH 5.8, containing 0.1 mM EDTA. The proteins linked to the carrier are separated by the stepwise gradient of the maleate buffer solution, pH 5.8, containing 0.04 M NaCl, 0.02 M-step increasing the salt concentration; the target fractions collection starts at the 0.06 M salt concentration. Chromatography temperature: 2 to 4°C. The target fraction is stage by stage ultra filtered at max. 8.0 kfg/cm$^2$ back pressure, through 5 kDa and 200 kDa retention potential materials; then desalinized, and diluted with the 50 M aspartate-NaOH solution, pH 7.4, down to the 1.0 mg/ml protein concentration, adding the polyoxyethylenesorbite monooleate (Tween 20) to the 0.01 mg/ml total concentration. The solution is sterilizing filtered, through a max. 0.22 $\mu$m mesh membrane filter. To characterize the resulting protein-polypeptide fraction the ultraviolet spectrophotometry, gel chromatography, electrophoresis in polyacrylamide gel, and amino acid analysis were used. The solution ultraviolet spectrum is read in the 250 to 350 nm wave length range, the maximum absorption is observed at $280\pm5$ nm. To determine the molecular mass of the complex forming proteins and polypeptides the following methods are used: gel chromatography with Superdex 75 (GE Healthcare) and denaturing SDS-Page electrophoresis in 12-percent polyacrylamide gel in comparison with standard marker proteins set. The column is calibrated with the protein standards, molecular masses range: 13 kDa to 250 kDa. The above methods have found that the drug contains the negatively charged faintly acid neutral proteins and polypeptides relating to the growth factor, differentiation factor, 5 to 200 kDa molecular mass signaling molecules; wherein at least 82% of the total protein mass has the 10 to 120 kDa molecular mass; and wherein a peak exists at the 284 nm wave length in the UV visible spectrum and bands exist in the pI 4.2 to 8.4 range at isoelectric focusing in a 5% polyacrylamide gel; and which drug contains a pharmaceutically acceptable diluent.

Resulting complex amino acid analysis (%) : Asp: 10.82 + 1.3; Thr: 5.4 + 0.9; Ser: 5.2 + 1.1; Glu: 16.2 + 1.9; Pro: 7.045 + 1.7; Gly: 5.2 + 0.8; Ala: 5.4 + 1.1; Val: 7.08 + 2.3; Met: 2.65 + 0.6; He: 4.45 + 0.8; Leu: 9.4 + 2.5; Tyr: 4.02 + 0.6; Phe: 4.8 + 1.1; Orn: 0.48 + 0.1; Lys: 8.48 + 2.3; His: 2.8 + 0.7; and Arg: 6.52 + 2.1.

**Example 8. Pharmaceutical composition production method.**

The obtained as of Example 1 porcine embryonic brain biologically active complex solution is diluted with the 50 mM glycin-phosphate buffer solution, containing, in total, 0.5% mannitol, 0.1% carmellose, 0.0005% Polysorbate 80, and disubstituted sodium phosphate to pH 8.0, to get the final total protein in resulting solution concentration within the 0.9 to 1.2 mg/ml range.

The resulting pharmaceutical composition is sterilizing filtered, through a max. 0.1 $\mu$m mesh membrane filter, dispensed in glass vials, and plugged.

The resulting pharmaceutical composition properties were assessed following the conventional experimental (preclinical) study of new pharmacological substances guidelines [5].

**Example 9. Pharmaceutical composition production method.**

The obtained as of Example 2 porcine embryonic brain biologically active complex solution is diluted with the 50 mM maleate buffer solution, pH 5.8, containing, in total, 0.3% lactic acid, 0.5% mannitol, and 0.1% carmellose, to 0.0005% Polysorbate 80, to get the final total protein in resulting solution concentration within the 0.9 to 1.2 mg/ml range.
The resulting pharmaceutical composition is sterilizing filtered, through a max. 0.1 μm mesh membrane filter, dispensed in glass vials, and plugged.

Example 10. Toxicity study

The toxicity was tested in the acute and subchronic experiments with the original pharmaceutical composition. Pharmaceutical form: sterile intranasal dosing spray, in 10 and 30 ml (0.1 mg/ml) glass or polymer vials; solution for injection: 5 ampoules 1 ml each (0.1 mg/ml), 5 ampoules 2 ml each (0.1 mg/ml), and 1 ampoule 2 ml (0.2 mg/ml). Maximum recommended daily doses: 0.4 mg endolumbarly, and 0.1 mg intravenously.

*Experimental conditions and methods*

The study purpose was to determine the original pharmaceutical composition tolerable, toxic, and lethal doses. The experiment used the both sexes mice, BALB/C line, 18 to 20 g body weight, intragastrically and intraperitoneally. Observation term: 14 days. The animals were received from nursery, certified. The animals were kept in standard condition, plastic cages, 10 animals each, and 10 animals in group. Feed: combined feed, fresh vegetables, curds. Free access to water and feed. Vivarium light: artificial. Air temperature: 18 to 20°C. The maximum possible for administration to mice dose was 0.2 ml, or 10 mg/kg, i.e., 600-fold daily therapeutic dose. The animals were withdrawn from experiment with ether narcosis; the animals were prosected; and the viscera were analyzed.

***Acute toxicity study results.***

*Mice*. Body weight: 18 to 20 grams. Administered dose: 0.2 ml, i.e., 10 mg/kg. Animals in group: 10. Two groups: intragastrical and intraperitoneal. Observation term: 14 days.
*Habitus.* To the end of experiment all the animals looked healthy. Hair: dense, white color. No differences between groups as of habitus.
*Behavior.* No differences between groups, normal.
*Death rate.* No deaths in any of the three groups.
*Body weight.* Six control weighings during experiment. No loss or growth revealed.
*Viscera.* No differences between groups as of viscera conditions. No viscera alterations or augmentation.

**Subacute (subchronic) toxicity study**

***Experimental conditions and methods***

The original pharmaceutical composition subchronic toxicity experimental study used the 200 to 250 grams body weight Wistar male and female rats. The animals were received from nursery. The animals were kept in standard condition, plastic cages, 5 animals each. Feed: cubed feed, fresh vegetables, curds. Free access to water and feed. Vivarium light: artificial. Air temperature: 18 to 20°C. Before experiment the animals were 10 days kept in quarantine. Ten animals (5 males and 5 females) in group. Groups segregation:

group 1 (control): intact animals;

group 2: original pharmaceutical composition, therapeutic dose, subcutaneous;

group 3: original pharmaceutical composition x 10, subcutaneous;

group 4: original pharmaceutical composition, intravenous, therapeutic dose; and

group 5: original pharmaceutical composition x 10, intravenous.

Observation term: 1 month administration, 1 month resorptive effect observation.

Toxicity assessment according to observation check list points:

- survival rate and habitus: (hair, eyes, ears, extremities, teeth);

- status and behavior (activity, walking, temperament, eating);

- body weight change (weighing before and at the end of the experiment); and

- physiologic functions (respiration, salivation, urination, excreta).

Before and at the end of the experiment the animals were blood tested: peripheral blood morphology (erythrocytes, leucocytes, thrombocytes counts, hemoglobin level); biochemistry (albumin, urea, creatinine, glucose levels); and activity of certain enzymes (aspartate aminotransferase, alanine aminotransferase, alkaline phosphatase, lactate dehydrogenase). The blood for hematologic study was taken from the tail vein. To count the blood corpuscles the Picoscale (Hungary) automatic counter was used. The hemoglobin level was determined by the cyanmethemoglobin method. To determine the biochemistry parameters the FP-901, Labsystems, Finland, automated system was used. After the chronic experiment completion the animals were sacrificed by narcosis overdose, for pathomorphological exams of the organs and tissues. The animals were prosected right after death, complete pathological study pattern. For the pathomorphological exams, the tissue samples were gelatinized and cryostatted to obtain the up to 5 $\mu$m thick slices. Then the tissues were fixed and hematoxylin-eosin stained. The microscopy used the Opton (Germany) microscope. The experimental data were compared with the control ones and counted using statistical software.

***Results of Study****. Macroscopic.*

*Survival rate:* All the experimental animals tolerated all the administration methods. No deaths were observed in either the experimental or control groups.

*Behavior:* No behavior (hyperactivity or hypoactivity) or status deviations of the experimental animals compared with the control ones were recorded. Muscular tone hypererethism: none.

*Habitus.* all animals were of medium finish; no atrophies or obesities. Hair: smooth and bright; no falloffs or fragility. Corneal haze, lacrimation, other pathologies: none. Earflaps: rose color, no scabs, no inflammations, no jerks. Teeth: regular color in all animals, no breaks.

*Functions:* respiration: quiet, regular rhythm, unobstructed in both experimental and control groups; salivation: no pathologies; urination: rate, volume, and color within the physiologic norm.

*Clinical: Body weight dynamics:* positive in all experimental groups. No firm differences between experimental and control groups.

*Hematology:* hemoglobin level, erythrocytes, leucocytes, and thrombocytes counts: within the physiologic norm in all groups.

*Biochemistry*: the peptide complex test dose did not affect the blood serum protein in the experimental animals; hence, no damaging effect of the tested drug on the liver protein-forming function. To reveal an eventual adverse hepatic effect, the aspartate aminotransferase, alanine aminotransferase, and alkaline phosphatase activity in the experimental animals blood serum was determined.

The data analysis showed no reliable alterations in the said blood serum enzymes activity.

*Pathomorphology: Macroscopic.*

In all the animals in all the groups at prosection: clean skin, moderately developed subcutaneous fat layer. Viscera arrangement: regular; no free liquid in either the pleural or abdominal cavities. Tracheal and bronchial lumen: free; mucosa: clean, wet, and lustrous. The pulmonary tissue in the experimental groups has a mode intense coloration than in the control ones, without, however, edema signs.

Myocardium on section: no lesions.

Tongue: clean. Buccal cavity and esophagus mucosas: no defects. Stomach and intestines: no traces of irritation. Liver: not enlarged.

The renal capsule is easily separable; the medullar and cortical substance on section are well distinguishable.

The spleen has a smooth capsule, grayish-brown color, no scrape on pulp.

Seminal glands and ovaries: no peculiarities.

Thymus: grayish color, no hemorrhages.

Thyroid gland: dense, with symmetric lobes.

Brain tunics: moderate blood filling, wet, lustrous. Brain matter: symmetric pattern on section.

The body weight indexes of the experimental animals had no valid differences with those of the control groups.

Microscopy: In all the studied groups: no pathomorphological alterations in the viscera (liver, kidneys, lungs, heart, spleen, stomach, large and small intestines), endocrine glands (thyroid, thymus, adrenal glands, pancreatic gland), reproductive organs (uterus, ovaries), lymph nodes, brain, skin, and nose and throat mucosas.

**Subchronic toxicity at intranasal administration.**

**Mature animals.**

The study used the mature female 2500 grams body weight Chinchilla rabbits. The animals were received from nursery. The animals were kept in standard metal cages, one animal per cage. Feed: cubed feed, fresh vegetables. Free access to water and feed. Vivarium light: artificial. Air temperature: 18 to 20°C. Three group, five animals in each. Groups segregation:

> group 1 (therapeutic dose): 0.1 mg/ml, 0.2 ml pharmaceutical composition, daily, 1/day;
> group 2: 0.1 mg/ml, 0.2 ml pharmaceutical composition, daily, 2/day; and
> group 3: intact animals.

Term of administration: 1 month.

Toxicity assessment according to observation check list points: survival rate, habitus, status, behavior, and body weight change (weighing before and at the end of the experiment). The local irritant effect during experiment was assessed from the nose mucosas alterations.

Before and at the end of the experiment the experimental animals were blood tested: peripheral blood morphology (erythrocytes, leucocytes, thrombocytes counts, hemoglobin level) and biochemistry (albumin, urea, creatinine, glucose levels); activity of certain enzymes (aspartate aminotransferase, alanine aminotransferase, alkaline phosphatase, lactate dehydrogenase). The blood to determine the above was taken from the auricular veins, 1.5 to 2.0 ml.

To count the blood corpuscles the Picoscale (Hungary) automatic counter was used. The hemoglobin level was determined by the cyanmethemoglobin method. To determine the biochemistry parameters the FP-901, Labsystems, Finland, automated system was used.

After the chronic experiment completion the animals were sacrificed by narcosis overdose. The animals were prosected right after death, complete pathological study pattern.

The morphometric assessment of the animal organs parameters used the Sartorius (Germany) scale; further on, the organs' masses and their standard deviations were calculated.

For the pathomorphological exams, the tissue samples were gelatinized and cryostatted to obtain the up to 5 $\mu$m thick slices. Then the tissues were fixed and hematoxylin-eosin stained. The microscopy used the Opton (Germany) microscope.

The experimental data were compared with the control ones and counted using statistical software.

**Results of Study**

*1. Visual.*

*Survival rate*: All the experimental animals tolerated all the administered doses of the pharmaceutical composition. No deaths were recorded.

*Behavior*: No hyperactivity or hypoactivity in the experimental animals compared with the control ones were observed. No muscular tone hypererethism was recorded in either the experimental or control animals. It was seen that the drug administration does not provoke discomfort in the animals.

*Habitus*: all animals, independently of the dose, were of medium finish; no atrophies or obesities. Hair: smooth and bright; no falloffs or fragility. Corneal haze, lacrimation, other pathologies: none. Earflaps: rose color, no scabs, no inflammations, no jerks. Teeth: regular color in all animals, no breaks.

*Functions*: respiration: quiet, regular rhythm, unobstructed in both experimental and control; salivation: no pathologies; urination: rate, volume, and color within the physiologic norm; and excreta: color and shape same as control.

*2. Clinical:*

*Body weight dynamics*: positive in all the experimental groups, no significant difference from intact control.

*Blood test:* hemoglobin level, erythrocytes, leucocytes, and thrombocytes counts: within the physiologic norm in all groups.

*Biochemistry:* blood glucose, alanine and asparagine aminotransferases activity normal in all groups. The protein concentration was not statistically different between the animals having received the pharmaceutical composition once and twice daily.

### 3. Pathomorphology:

In all the studied groups: no pathomorphological alterations in the viscera (liver, kidneys, lungs, heart, spleen, stomach, large and small intestines), endocrine glands (thyroid, thymus, adrenal glands, pancreatic gland), reproductive organs (uterus, ovaries), lymph nodes, brain, skin, nose and throat mucosas.

Thus, the pharmaceutical composition nasal form subchronic toxicity was tested. The spray subchronic test did not reveal either any significant alterations of the experimental animals viscera, or local irritant effect. The subchronic toxicity study used the clinical methods, conventional methods for the blood hematology and biochemistry, and viscera and nasal mucosas histology.

### Subchronic toxicity study in immature rabbits at intranasal administration.

The study used the 3-week age immature 300 to 500 grams body weight Chinchilla rabbits. The drug was administered daily, 1 or 2/day, single spray, during 2 weeks. The animals were received from nursery. The animals were kept in standard metal cages, one animal per cage. Feed: cubed feed, fresh vegetables. Free access to water and feed. Vivarium light: artificial. Air temperature: 18 to 20°C. Three groups, five animals in each.

Groups segregation:

group 1 (therapeutic dose): 0.1 mg/ml, 0.2 ml pharmaceutical composition, daily, 1/day;
group 2: 0.1 mg/ml, 0.2 ml pharmaceutical composition, daily, 2/day; and
group 3: intact animals.

Term of administration: 2 weeks.

Toxicity assessment according to observation check list points: survival rate, habitus, status, behavior, and body weight change (weighing before and at the end of the experiment). The local irritant effect during experiment was assessed from the nose mucosas alterations.

Before and at the end of the experiment the animals were blood tested: peripheral blood morphology (erythrocytes, leucocytes, thrombocytes counts, hemoglobin level) and biochemistry (albumin, urea, creatinine, glucose levels); activity of certain enzymes (aspartate aminotransferase, alanine aminotransferase, alkaline phosphatase, lactate dehydrogenase). The blood to determine the above was taken from the auricular veins, 1.5 to 2.0 ml. To count the blood corpuscles the Picoscale (Hungary) automatic counter was used. The hemoglobin level was determined by the cyanmethemoglobin method. To determine the biochemistry parameters the FP-901, Labsystems, Finland, automated system was used.

After the chronic experiment completion the animals were sacrificed by narcosis overdose. The animals were prosected right after death, complete pathological study pattern.

The morphometric assessment of the animal organs parameters used the Sartorius (Germany) scale; further on, the organs' masses and their standard deviations were calculated.

For the pathomorphological exams, the tissue samples were gelatinized and cryostatted to obtain the up to 5 $\mu$m thick slices. Then the tissues were fixed and hematoxylin-eosin stained. The microscopy used the Opton (Germany) microscope.

The experimental data were compared with the control ones and counted using statistical software.

### Results of Study

#### Assessment of irritant effect on nasal mucosa.

*Intravital biomicroscopy*

During the whole observation term, the animal behavior remained normal; no ooze from the nasal passages or scabs were recorded. The drops provoked in the rabbits the sneezing reflex, stopping in 1 to 2 minutes. At the instillation of drops, both the therapeutic group and the overdose group rabbits had the mechanical irritation effect, expressed in the sneezing reflex, nose jerks; the animals rubbed their noses with their paws.

*Control intact group.* Mucosa: pale rose color, no edemas or ooze. The otolaryngologic speculum observation revealed no lesions on the epithelium. Nasal mucous secretion: transparent, hydrous. Reflexes: within the physiologic norm.

Confocal microscopy: epithelial layer of densely packed structure of clearly differentiated cells. The cells are connected by gap junctions.

*Therapeutic dose group.* Clean nose mucosa during the whole observation term: weak rose color, no oozes or edemas. Mucosa vessels: unchanged. The epithelium state was assessed with the slit illumination. No lesions were revealed.

*X 10 group.* No lesions were revealed.

*Clinical and biochemical*

*Blood test*: hemoglobin level, erythrocytes, leucocytes, and thrombocytes counts: within the physiologic norm in all groups. The experimental groups results showed no significant differences from the control intact groups and comparison drug.

*Biochemistry*: blood glucose, alanine and asparagine aminotransferases activity normal in all groups.

**Conclusion.** The original pharmaceutical composition was subchronic toxicity tested in experimental immature animals.

It was revealed that the substance intranasal administration, in both the therapeutic and ten-fold therapeutic doses, in the course of 2 weeks, provokes no viscera alteration and gives no toxic effect on the blood system. No local irritant effect was revealed.

**Example 11. Pharmaceutical composition mutagenicity.**

To assess the claimed protein-polypeptide pharmaceutical composition mutagenicity, the following set of tests was used:

- Ames Salmonella/microsome gene mutagenicity assay using the TA 97, TA 98, and TA 100 Salmonella typhimurium strains as test objects; and
- micronuclei in murine bone marrow cells.

A protein-polypeptide complex sample was tested, in form of transparent liquid sterile packed in a dark glass vial, 20 ml, 0.1 mg/ml substance.

**Ames Test Protein-Polypeptide Pharmaceutical Composition Mutagenicity Assessment.**

The Salmonella typhimurium mutagenicity assay is a bacterial test system to account for the histidine prototrophy gene mutations under effect of the chemical compounds and/or their metabolites, inducing the mutations of the base substitution or reading frame shift in organism genome type. The mutagens inducing the base pairs substitution are the agents provoking the base pairs substitution mutations in the DNA molecule. The mutagens inducing the genetic code reading frame shift mutations are the agents provoking the addition or deficit of single or multiple base pairs in the DNA molecule.

The method's purpose is to reveal the capability of the pharmaceutical substances or their metabolites to induce the gene mutations in the Salmonella typhimurium indicator strains. The bacteria are treated with a compound under test with (SM+) or without (SM-) metabolic activation system. After a certain time incubation, the different test strains revertant colonies number is compared with that of the negative control spontaneous revertant (untreated or solvent treated cultures). If the compound under test and/or its metabolites have mutagenic activity, they will induce the reverse mutations from histidine auxotrophy to prototrophy in the Salmonella typhimurium histidine-dependent strains.

The tests used a set of the Salmonella typhimurium indicator strains allowing to register the genetic code reading frame shift (TA 98 and TA 97) base pair substitution (TA 100) mutations. The strains originated from the Russian National Collection of Industrial Microorganisms, Research Institute for Genetics and Selection of Industrial Microorganisms, Scientific Center of Russian Federation.

The bacterial cultures handling Rules of procedure, the strains genotype checks, their museum keeping rules, the experimental utensils, reagents, nutrient media and solutions, rat liver homogenate getting, activating mix compounding, and statistical analysis methods complied with the standards described in detail in the relevant literature.

The metabolic activation used the S9 fraction of the Wistar male rats liver; 5 days before sacrifice the rats received the sovol microsomal enzymes inductor (300 mg/kg, single, intraperitoneally). To control the metabolic activation system the ethidium bromide (10 $\mu$g/dish), TA 98 strain, SM+, was used.

The 0.1 mg/ml protein sterile protein-polypeptide complex solution was examined: 1.0 and 0.3 ml/dish mother solution and three 10X dilution in physiological solution (0.1 ml/dish). The substance test doses were 300, 100, 10, 1, and 0.1 μg/dish.

The experiment had positive controls; for them the mutation-inducing substances in relevant microorganisms strains with or without activation were used. The no-activation variants used the sodium azide, 10 μg/dish, TA 100 strain, SM-; 2, 7-diamino-4, 9-dioxy-5, 10-dioxo-4, 5, 9, 10-tetrahydro-4, 9-diazopyrene (DDDTDP), 10 μg/dish, TA 98 strain, SM-; and 9-aminocaridine (9AA), 50 μg/dish, TA 97 strain, SM-. To control the metabolic activation system activity the ethidium bromide (10 μg/dish), TA 98 strain, SM+, was used. The negative control used the physiological solution (0.1 ml/dish).

The selective semi-enriched agar (0.7%) in test tubes was melted in water bath at 100°C; then it was placed into the 45-46°C thermostatted water bath.

First, 0.1 ml of the relevantly diluted sample, then 0.1 ml of the bacterial suspension and 0.5 ml of the microsomal activating mix (metabolic activation variant) were put into the agar test tubes. Then the tube content was quickly blended and discharged onto the lower minimal agar in the Petri dishes. The microsomal activating mix and semi-liquid agar reserve introduction time per dish did not exceed 10 to 15 seconds. The dishes were left for 30 to 40 minutes at ambient temperature; after the first agar gelation they were put into a 37°• thermostat. The results were accounted after a 48-hour incubation.

The no metabolic activation system (SM-) and metabolic activation system (SM+) variants were experimented in parallel. In the SM- variant the direct mutagens (drugs mutagenic due to the compound initial structure activity) effect may be observed. As of the effect of the promutagens, i.e., the compounds whose effect associates with the mutagenic metabolites formation, this may be accounted when comparing the results of the SM- and SM+ compounds tests analysis data. Every control and experimental variants used 2 dishes each. The mutagenic effect was considered significative when the mean number of the revertants colonies per dish in experiment exceeded the control one 2- and more fold. The experimental results were taken into account at the condition of standard response in all the positive and negative control variants. The revertants colonies numbers in the solvent control, SM- and SM+ variants, were within the spontaneous level fluctuations range for the given strains. The strains response to the standard mutagens was within the common levels range.

The examined with all concentrations protein-polypeptide complex did not show any mutagenic effect for the TA 100, TA 98, and TA 97 strains, with and without metabolic activation system.

CONCLUSION: the protein-polypeptide pharmaceutical composition is unable to induce gene mutations with the Salmonella typhimurium test strains within all the experimental concentrations range.

**Micronuclear Test Protein-Polypeptide Composition Mutagenicity Assessment in Mammalian Cells.**

The cytogenetic activity is the capacity of a substance to provoke structural and quantitative chromosomal abnormalities in the somatic and embryonic cells.

The micronuclei are the small size DNA-containing formations consisting of the chromosomal acentric fragments or lagged in ana-telophase chromosomes. At the telophase stage these fragments may get into the daughter cells nuclei or form individual or multiple micronuclei in cytoplasm.

The Study purpose was to reveal and quantitatively assess the cytogenetic activity (mutagenicity) of the pharmacologic drugs in the mammalian bone marrow polychromatophils. The method's base is the micronuclei cells microscopic registration.

The experimental used the male and female F1(CBAx C57Bl6/J) hybrid mice, two-month age, 18 to 20 grams body weight. Every group included 6 animals. The animals were kept under the 12-hour light regime, with freely available water and food. The single and five fold administration protein-polypeptide complex mutagenicity assessment animal experiments were in compliance with the official protocols. Three experimental series with relevant controls were performed: therapeutic dosage: males, single administration; subtoxic dosage: males, single administration; and therapeutic dosage: males and females, five fold administration.

Taking into account that the possible biologically active protein-polypeptide pharmaceutical composition clinical administration method may be intravenous or endolumbar, this Study used the intraperitoneal one with mice. The protein-polypeptide complex mutagenicity assessment dose was defined on the basis of the maximum recommended daily therapeutic dose (TD) for man. The 2 ml at 0.1 mg/ml concentration IV administration is recommended. The therapeutic dose for man, including infant, may be 0.05 mg/kg/day. To calculate the mice experimental dose, the conversion factor is recommended to take into account the human and murine body surface area to body weight ratio. In our study it was 8.33. Hence, for the murine therapeutic dose the approximately 8.33 human therapeutic dose was taken (0.42 mg/kg).

For the subtoxic dose the maximum possible experimental one was used, 10 mg/kg, as the substance concentration was 0.1 mg/ml; the conventional total substance administered to mice is 0.1 ml per 10 g body weight. Hence, calculated for man, the maximum tested dose was 1.7 mg/kg.

Seven groups of animals, four experimental and three control ones, were formed. The 0.1 mg/kg substance was five times, at 24-hour intervals, administered to both the male and female animals. In all the groups the animals were sacrificed 6 hours after the last protein-polypeptide complex administration.

In two other experimental groups, the protein-polypeptide complex was single administered to the males, at 0.2 mg/kg and 0.5 mg/kg.

For negative control, the physiological solution was used. It was administered intraperitoneally to the males and females of the two control groups, the times and volumes were the same as for the experimental. Positive control: 20 mg/kg cyclophosphamide dissolved ex tempore in physiological solution, single intraperitoneal administration, 24 hours before sacrifice.

The bone marrow cells preparations for the micronuclei account were made in conventional manner, in accordance with the "Micronuclear Test Environmental Factors Mutagenicity Assessment in Mammalian Organs Cells" Guidelines. The sacrifice method was the cervical dislocation 6 hours after last drug administration. Both femoral bones were extracted, muscles were then removed. To wash out the bone marrow the bovine embryonic serum (NPP PanECO) was used. The bone marrow from the both femoral bones was extracted into a microtube with a syringe. The suspension was centrifuged at 1000 rpm, 5 minutes; the supernatant was removed, the sediment was carefully resuspended. A drop of the suspension was used to prepare the smear then dried in air. The smear was then methanol fixed during 3 minutes. For staining the Romanowsky-Giemsa method was used. 2000 polychromatophils per animal were then analyzed with coded preparations. At the 500 erythrocytes count the polychromatophil to normochromatic erythrocytes ratio was determined. Analysis finished, the preparations were coded.

The positive result criterion is the reproducible and statistically significant growth of the micronuclei polychromatophil erythrocytes number in at least one experimental group compared with the control one. The positive result indicates that the substance induces the chromosomal damages and/or cells mitotic apparatus damages in experimental animals.

The micronuclei account results statistical treatment used the X2 criterion experimental to control series comparison; the polychromatophils proportion intergroup comparison used the Mann-Whitney criterion.

Mice bone marrow micronuclei polychromatophils account results: in no experimental variants the protein-polypeptide pharmaceutical composition induced the mice bone marrow micronuclei polychromatophils proportion augmentation as compared between the experimental and relevant control series. The 20 mg/kg cyclophosphamide (positive control) intraperitoneally in male mice provoked 19.6-fold higher micronuclei cells (62.1‰ vs. 3.16‰ in control, $P < 0.001$). The polychromatophil erythrocytes share in the total bone marrow erythrocytes was at approximately the same level for both the experimental and control groups; hence, no toxic effect of the substance in tested doses on hematogenesis. Under the positive control (cyclophosphamide) the polychromatophil to normochromatic erythrocytes ratio shift toward the latter ones was recorded (compared with control, $P < 0.005$).

Thus, the micronuclei induction test in the mice bone marrow micronuclei polychromatophils at one and five fold 0.42 mg/kg body weight intraperitoneal administration to male and female animals (corresponding, as recounted, to the daily therapeutic dose for human) revealed no protein-polypeptide complex mutagenic activity. No mutagenic activity was revealed for the maximum possible, 10 mg/kg, single protein-polypeptide pharmaceutical composition administration in males. The protein-polypeptide complex showed no toxic effect as of the "polychromatophil erythrocytes proportion" parameter.

**Conclusion for the protein-polypeptide pharmaceutical composition mutagenicity assessment.**

The protein-polypeptide pharmaceutical composition showed no mutagenic activity in either the Ames Salmonella/microsomes test or the mice bone marrow cells micronuclei induction test performed in accordance with the Guidelines for Experimental (Preclinical) Study of New Pharmacological Substances.

**Example 12. Effect of pharmaceutical composition on rats survival rate under brain ischemic damage at both carotid arteries ligation (incomplete global ischemia of brain).**

The experimental used the Wistar male rats, 180 to 250 g body weight, under irreversible bilateral occlusion of the common carotid arteries [3-5]. The laboratory animals received from nursery were 5 days kept in quarantine. All the veterinary manipulations were aseptic, to keep the experimental animals status. The experimental animals were kept in standard conditions [6]. The rats received the ether (chemically pure diethyl ether, OAO "Medhimprom") anesthesia; then a 8 to 10 cm long skin discission was made in the neck region, along the median line, to access the carotid arteries. The both carotid arteries were exposed, separating the vagus and sympathetic nerves. The silk threads (Silk blk 17 x 45 cm/• 3/USP2/ Non needled/ Ethicon Johnson & Johnson, cat. No W203) were pulled under the vessels; then, together with an assistant, the both carotid arteries were single-step ligated with triple friction knot. The skin discission was then sutured with the surgical silk (Silk blk 100 cm/• 5/Sgle armed KP-3USP2/ Non needled/ Ethicon Johnson & Johnson, cat. No W794), one or two surgical stitches, with further wound treatment with the 5% iodine tincture. The physiological solution or pharmaceutical composition were administered intraperitoneally. The total manipulations time was 8 to 10 minutes; then the animals were placed into cage. Three groups of animals were used:

- **group 1:** control animals, physiological solution with sterile disposable insulin syringe: 0.5 ml/rat in 1 hour and 0.5 ml/rat in 5 hours after carotid arteries ligation;

- **group 2:** sham operated animals: same manipulations as in control but without carotid arteries ligation; physiological solution 0.5 ml/rat in 1 hour and 0.5 ml/rat in 5 hours; and

- **group 3:** animals after carotid arteries ligation; biologically active substance: 0.5 ml/rat 0.1% solution (0.2 mg/kg) in 1 hour and 0.5 ml/rat in 5 hours.

The survival rate was followed up immediately after the procedure and in the course of the first day, i.e., the final survival rate was fixed in 24 hours. The drug biological activity in form of the brain protection under incomplete global ischemia at single-step carotid arteries ligation was assessed as follows:

- **expressed**: survival rate: > 80% rats;

- **median expressed**: survival rate: 70% to 80% rats; and

- **unexpressed**: survival rate: < 70% rats.

The data statistical processing used the Fischer exact test, accounting for the comparisons multiplicity.

The experiments showed that the greatest number, i.e., 11 of 24, of the animals in control died within the 1st hour after the carotid arteries ligation; 4 more animals died within the next 24 hours; in total, 15 rats of 24 died (63%, see Table 1). Among the sham operated animals 2 (17%) rats died immediately after the operation. The pharmaceutical composition 0.2 mg/kg (0.5 ml 0.1% solution) intraperitoneally in 1 and 5 hours provoked death in only two animals: 34/36 rats survived. The compared to control survival rate augmentation is 95%. Table 6 shows the obtained data.

**Table 6. Effect of pharmaceutical composition on rats survival rate under ligation of both carotid arteries**

| Groups of animals | Expressed bioactivity | |
|---|---|---|
| | Survived | Died |
| Group 1<br>Control: 24 rats | 9 of 24<br>(37%) | 15 of 24<br>63% |
| Group 2<br>Sham operated<br>12 rats | 10 of 12*<br>(83%) | 2 of 12<br>(17)% |
| Group 3<br>Pharmaceutical composition 0.2 mg/kg<br>36 rats | 34 of 36*<br>(95%) | 2 of 36<br>(5%) |
| * -P • 0.01 compared with control. | | |

Hence, the pharmaceutical composition ensures a statistically significant animals survival rate augmentation compared with control under incomplete global ischemia of brain at single-step both carotid arteries ligation.

The Study revealed that the claimed pharmaceutical composition has the expressed neuroprotective and neurometabolic effects stimulating the nervous tissue physiologic and reparative regeneration; its biological activity is higher than that of the Cerebrolysin reference drug.

The claimed pharmaceutical composition may find application in the treatment of the vascular, traumatic, toxic, hypoxic, and autoimmune genesis central and peripheral nervous system diseases.

**Example 13. Study of pharmacologic composition effect on local cerebral blood flow in anesthetized rμats brain cortex under global transient ischemia**.

The experimental used the anesthetized Wistar line male rats, 220 to 250 grams body weight, natural respiration. The anesthesia used the urethane (Sigma Aldrich Chemie GmbH, Germany), 1400 mg/kg, intraperitoneally.

**Technique**. The brain blood circulation in animals was assessed using the rats brain cortex local blood flow Doppler laser flowmetry records. The local blood flow in the rats brain cortex parietal region was recorded with the ALF-21 flowmeter (Transonic System Inc., USA). For this, the 0.8 mm diameter flowmeter needle sensor was installed in the rat brain cortex parietal region with the help of a micromanipulator and beam. At the same time, the arterial blood pressure fluctuations were recorded using a polyethylene catheter preinserted into the femoral artery. The blood flow and arterial blood pressure readings were recorded by the BIOPAK (USA) polygraph and personal computer. The 0.2 mg/kg (0.5 ml 0.1% solution) pharmacologic composition was administered into the femoral vein via the polyethylene catheter.

The global transient ischemia model is widely used to study both the cerebral blood flow disturbances and the different post-ischemic brain function biochemical alterations. The global transient ischemia in rats was provoked by the 10-minute occlusion of the both common carotid arteries with the help of special clamps; at the same time, the arterial pressure was reduced down to 40 to 50 mm Hg by pumping out the arterial blood via the polyethylene catheter preinserted into femoral artery. In 10 minutes, the clamps were removed from the carotid arteries; the earlier taken blood was returned to the animal [3-5].

The test results were variance analysis treated using for the remeasurements the disperse analysis and the Dannett's multiple comparison test and Biostat software.

**Results of Study**

The experimental showed that the rats brain cortex global transient ischemia provokes the local brain blood flow downswing, which downswing is, in average, $6.1\pm1.0$ RVU or $82\pm2.2\%$ of the initial level. Having removed the clamps from the carotid arteries, and reinfused the blood, a blood flow upswing is observes, which gradually reduces, and, in 30 minutes after reperfusion, in average, $25\pm2.8$ RVU or $23\pm2.4\%$ of the initial level ($p < 0.05$).

The 0.2 mg/kg 0.1% (0.5 ml 0.1% solution) pharmacologic composition administered intravenously 30 minutes after reperfusion induced, in most cases, already 10 minutes after administration, the rats brain cortex local blood flow augmentation by, in average, $12\pm3.3\%$ (7/10 experiments). The start of the statistically significant augmentation of the local cerebral blood flow was observed on the 50th minute, the average augmentation  was $39\pm9.2\%$, and such augmentation continued until the 90th minute of observation (see Table 2). It should be noted here that in the physiological solution controls the local cerebral blood flow level remains practically the same.

The arterial blood pressure was measured in parallel with the cerebral blood flow. The experimental showed that 10 minutes after the pharmacologic substance administration to rats the gradual arterial blood pressure decrease was observed; the arterial blood pressure decreased in 50 minutes by, in average, $15\pm3.4\%$; and this hypotension remained up to the experiment completion (see Table).

It should be noted that the physiological solution controls also showed a small arterial pressure decrease, by, in average, 18%; hence, the pharmacologic substance has no significant effect on the arterial pressure level.

**Conclusion**

Hence, the Study showed that the pharmacologic substance improves the experimental animals survival rate under carotid arteries complete ligation. At the same time, the pharmacologic substance increases the rats brain cortex local blood flow, reduced after the brain global transient ischemia; to the end of the experimental, this flow is greater than the pre-ischemia control. The ligated carotid arteries animals survival rate augmentation under effect of the pharmacologic substance may be due to its capacity to improve the experimental animals brain cortex blood supply.

**Example 14. Pharmaceutical composition neuroprotective effects at *in vitro* glutamate toxicity.**

The in vitro test of the biologically active protein-polypeptide complex pharmaceutical composition neuroprotective capacities at glutamate toxicity used the 7-day cell cultures: Wistar line rats cerebellar granule cells obtained in enzymatic mechanic dissociation. Brain tissue dissociation method: the extracted cerebella were placed in a plastic Petri dish filled with phosphate buffer; then these were washed with the same solution several times and fragmented. The tissue fragments were 15-minute incubated at 37°C in the phosphate buffer based enzymatic mix with 0.05% trypsin and 0.02% EDTA. After incubation the tissue was washed twice in phosphate buffers and once in cultivation medium; then it was mechanically dissociated in the cultivation medium. The nutrient medium contained: 10% bovine embryonic serum, 2 mM glutamine, and 10 mM • • • • S buffer, pH 7.2-7.4. The cells suspension was 1-minute centrifuged at 1000 rpm; the supernatant was discharged; the sediment was resuspended in the nutrient medium where the K+ concentration had been brought to 25 mM, which has a trophic effect on the cerebellar granule cells. The cultivation went on in the 96-socket plastic cameras. Into every socket, 0.1 ml cells suspension was added. The cultures developed in a $CO_2$ incubator, at 35.5°C, 98% RH.

To the 7th cultivation day, upon the glutamate receptors maturity, the 15-minute glutamate toxic treatment (25, 37, 50 μM, 15 minutes) was performed, in the balanced saline solution (mM: 154 NaCl, 25 KCl, 2.3 $CaCl_2$, 1 $MgCl_2$ 3.6 $NaHCO_3$, 0.35 $Na_2HPO_4$, 10 HEPES (pH 7.6)); then the preparation was washed twice; the third solution contained the pharmaceutical composition (0.05 and 0.1 mg/ml). Control (same volume) : • $_2$•, glycin buffer, and bovine serum albumin (0.15 mg/ml). All was then 4-hour $CO_2$-incubated to develop the neurodegeneration. Then the mix was 20-minute fixed with the 2:7:1 formalin- alcohol- acetic acid mix, stained with the trypan blue, and filled with glycerol; the photos were shot by a digital camera.

The neurons viability was assessed by counting the normal morphology neurons on the fixed trypan blue stained preparations encapsulated in glycerol. Every point used 3 sister cultures; the cells were counted in 5 culture visual fields, x 40 lens. The survival rate was calculated as percentage of control.

The protective effect is more illustrative having calculated the protection efficiency factor (PEF) from the formula:

$$PEF = (N• - N•)/N• \times 100\%,$$

where: NO: mean percentage of the neurons loss due to the noci-influence (here, glutamate) in the • 2• addition cultures; and NB: mean percentage of the neurons loss in the neuron pharmaceutical composition addition cultures. This parameter allows for comparing the efficiencies of different substances with different ischemia models.

## Results of Study

**Series 1**. The cerebellum dissociated cultures consist of the neurons and neuroglials. The neuronal population is represented by the granule cells as the other larger size cerebellum neurons are already differentiated to the dissociation moment, and, hence, do not survive the procedure. The granule cells form the largest class of the brain neurons; they are morphologically and neurochemically homogeneous. To day 7 of cultivation the glutamate receptors become mature.

The 3 to 4-hour exposure to the balanced saline solution with the glycin buffer (GB), bovine serum albumin (BSA), and 0.05 and 0.1 mg/ml pharmaceutical composition added led to no change of the living neurons number compared with control.

The 25, 37, and 50 μM (15 minutes) glutamate provoked the dose-dependent death of the murine cultivated cerebellar granule cells.

To reveal the protective effects of different substances the glutamate induced death of cells must not exceed 70%. Thus, the neuroprotective experimental used the 25 μM glutamate concentration. Both the 0.1 and 0.05 mg/ml pharmaceutical compositions improve the protective effect, increasing the cerebellar granule cells survival rate from 54.97+6.5% to 72.76+9.7%, respectively. However, this effect is more expressed at a lower concentration.

In our event, for the 0.05 mg/ml pharmaceutical composition, PEF = (45.03-27.24) /45.03 • 100% = 39.51%; 0.1 mg/ml: PEF = (45.03-37.85) /45.03 • 100% = 15.94%.

The glycin buffer and bovine serum albumin added in the post-glutamate period did not change the glutamate toxicity. In whole, the first experimental series counted about 2, 000 cells from 60 sister cultures.

**Series 2**. The second series used the same experimental techniques and patterns as the first one, except that the pharmaceutical composition used two, 0.05 • 0.01 mg/ml, concentrations, and the Cerebrolysin in the same concentrations was used for reference drug. The control calculation data show that the 3 to 4-hour exposure to the balanced saline solution with the 0.05 and 0.01 mg/ml glycin buffer, pharmaceutical composition, and Cerebrolysin additions did not lead to change of the living neurons number compared with control.

The 25, 37, and 50 μM (15 minutes) glutamate provoked the dose-dependent death of the murine cultivated cerebellar granule cells. To reveal the protective effects of different substances the glutamate induced death of cells must not exceed 70%. Thus, the neuroprotective experimental used the 25 μM glutamate concentration. The observed similar dose dependent surviving neurons score in function of the glutamate concentration at 35 and 50 μM indicated the receptors saturation. The pharmaceutical composition had a reliable protective effect, improving the cerebellar granule cells survival rate This effect if dose dependent: maximum protection at maximum biologically active protein-polypeptide complex concentration in pharmaceutical composition (0.05 mg/ml). Here, REF =(45-20) /45 • 100%=55.6%.

For 0.01 mg/ml: PEF = (45-36) /45 • 100% = 20%.

The tested dosages Cerebrolysin had no reliable protective effect (PEF = (45-38) /45 • 100% = 15.6%, at 0.05 mg/ml; and PEF = (45-42) /45 • 100% = 6.7%, at 0.01 mg/ml).

The post-glutamate period added glycin buffer even increased somehow (non-reliable) the glutamate toxicity.

**Pharmaceutical composition aggregate protective effect as of the experimental series:**

The above results of individual experiments show that the pharmaceutical composition protective effect after the toxic exposure to glutamate is stable, experiment-to-experiment reproducible. The drug itself showed no toxic effect in any experiment. Neurons death rate and protection factors: 3 hours after the 25 $\mu$M glutamate exposure 41.49+4% neurons were lost; if after the glutamate exposure the balanced saline solution had the 0.5 or 0.1 mg/ml pharmaceutical composition the cultivated cerebellar neurons death rate went down to the respective 30.88+4.3 and 21.98+4.37. With this, this glutamate toxicity reduction at the 0.5 or 0.1 mg/ml pharmaceutical composition introduction after lesion was reliable (n = 45, P < 0.05, Anova tow-way test with Benferroni post-test). Moreover, for the 0.1 mg/ml pharmaceutical composition, the trend was well expressed.

Protection efficiency factors (PEFs) for various concentrations of pharmaceutical composition:

$$PEF\ (0.01\ mg/ml) = (41.49\text{-}30.88)/41.49 \cdot 100\% = 25.57\%.$$

$$PEF\ (0.05\ mg/ml) = (41.49\text{-}21.23)/41.49 \cdot 100\% = 48.83\%.$$

$$PEF\ (0.01\ mg/ml) = (41.49\text{-}21.98)/41.49 \cdot 100\% = 47.02\%.$$

It should be noted that the above 80% or below 30% glutamate toxicity experimental results were neglected.

The tested dosages Cerebrolysin had no reliable protective effect (PEF = (45-38) /45 • 100% = 15.6%, at 0.05 mg/ml; and PEF = (45-42) /45 • 100% = 6.7%, at 0.01 mg/ml).

The post-glutamate period added glycin buffer even increased somehow (non-reliable) the glutamate toxicity.

**Example 15. Study of pharmaceutical composition pharmacologic effects mechanisms.**

The pharmaceutical composition specific effect on the excitatory and inhibitory neurotransmitters concentrations dynamics was studied in vitro, in the 7-day cerebellar granule cells cultures intercellular fluid, under glutamate cytotoxic effect. The drugs affecting the glutamate release (Lubelizil, BW619C89), are known to be efficient neuroprotectors.

**Technique**. The assay was taken of 50 $\mu$l of the cerebellar granule cells cultivation medium, before and after the glutamate toxic exposure, and pharmaceutical composition, glycin-phosphate buffer, and/or physiological solution addition. The resulting media samples were frozen at 20°C, coded, and sent to laboratory to measure the neurotransmitters concentrations in the high efficiency liquid chromatography with electrochemical detection. The LC-4B (• AS, USA) electrochemical detector at +850 mV potential on glass carbon electrode vs. reference Ag/AgCl electrode was used. The sodium phosphate 0.05 M buffer with 0.025 mM EDTA and 5% acetonitrile served as mobile phase. To derive the amino acids, to 25 $\mu$l perfusate, 25 $\mu$l 0.01 mg/ml L-homoserine internal standard in 0.2 n NaOH and 10 $\mu$l #-phtalal-dehydsulfite agent in 0.1 M borate buffer (pH 9.5) were added. For standard, the solution was used containing the 0.01 mmol/l amino acids mixture in 0.2 n $HClO_4$ and 0.2 mg/ml glutamate in 0.1 n $HClO_4$. After the 15-minute 37°C incubation, 25 $\mu$l of the mix were applied onto the Agilent Hypersil ODS 5 $\mu$m, 4.6 x 250 (5 $\mu$l loop volume) column of the Agilent 1100 (USA) chromatograph. The amino acids concentration was calculated with the Chemstation Agilent (USA) software; the end result was expressed in nM/mg of tissue in 2 minutes.

The results were statistically analyzed by the Biostat software, using the non-parametric criteria (Wilcoxon-Mann-Whitney U-test).

The mobile phase consisting of 0.069 M anhydrous $KH_2PO_4$ (Fluka), 0.27 M citric acid monohydrate (Fluka), 0.27 m• ethylenediaminotetraacetic acid sodium salt (Sigma), and 1.9 m• sodium octylsulfonate (ion-pair reagent) (Diapharm) was prepared as follows: the batch weight was diluted in 920 ml deionized water, pH was brought to 3.15 (pH-340 pH-meter), then 80 ml, i.e., 1.528 M acetonitrile (Merck) was added. The solution was vacuum filtered (0.2 $\mu$m microfilter) under 20 to 40 mm Hg pressure. Before the biochemical experiments the mobile phase was 40 to 50-second degasified under vacuum with simultaneous ultrasonic bath treatment (Serga, Russia).

**Results**: the 0.05 and 0.1 mg pharmaceutical composition reliably and dose-dependently augmented the glutamate, glycin, gamma-aminobutyric acid, taurin, and aspartate extracellular release (see Table).

**Table 7.**

| Experimental series | Tested neurotransmitters (μMol/l) | | | | |
|---|---|---|---|---|---|
| | **Asp** | **Glu** | **Gly** | **GABA** | **Tau** |
| Control | **0.0008**<br>+ 0.00015 | **0.00014**<br>+ 0.00067 | **0.011**<br>+ 0.0048 | **0.00023**<br>+ 0.00013 | **0.038**<br>+ 0.0091 |
| 0.05 mg pharmaceutical composition | **0.0011**<br>+ 0.00052 | **0.0003**<br>+ 0.000083 | **0.56**<br>+ 0.224 | **0.00047**<br>+ 0.0003 | **0.0635**<br>+ 0.029 |
| 0.1 mg pharmaceutical composition | **0.0011**<br>+ 0.0005 | **0.00034**<br>+ 0.00012 | **0.702**<br>+ 0.39 | **0.0004**<br>+ 0.00022 | **0.075**<br>+ 0.034 |
| Control with 25 μM glutamate toxicity | **0.0009**<br>+ 0.0004 | **0.00021**<br>+ 0.00013 | **0.0516**<br>+ 0.0417 | **0.00021**<br>+ 0.00012 | **0.0457**<br>+ 0.02023 |
| 0.05 mg pharmaceutical composition with 25 μM glutamate toxicity | **0.00075**<br>+ 0.000067 | **0.00027**<br>+ 0.00006 | **0.43341**<br>+ 0.22624 | **0.00037**<br>+ 0.00016 | **0.0651**<br>+ 0.01613 |
| 0.1 mg pharmaceutical composition with 25 μM glutamate toxicity | **0.0007**<br>+ 0.00002 | **0.00026**<br>+ 0.000153 | **0.7398**<br>+ 0.24536 | **0.00056**<br>+ 0.00033 | **0.07977**<br>+ 0.017958 |

At the 0.05 mg pharmaceutical composition concentration the neurotransmitters release level was by 20% higher ($p < 0.05$), at 0.1 mg the also reliable augmentation was 28%. After the 25 μM glutamate toxicity, and 0.05 mg and 0.1 mg pharmaceutical composition, the dose-dependent GABA, as inhibitory neurotransmitter, augmentation was observed; it made 90% and 160%, respectively, in relation to the control (• < 0.01). The taurin and glycin concentrations dose dependently grew to the same level, both with and without glutamate toxicity. The excitatory neurotransmitters, aspartate and glutamate, at glutamate toxicity and pharmaceutical composition reduced to the control for aspartate ($p < 0.05$) and remained unchanged with glutamate, without certain distinction from its effect without glutamate toxicity.

Hence, the pharmaceutical composition physiologic effect showed itself in the higher glycin, glutamate, taurin, and GABA concentrations. This effect intensity depended on the pharmaceutical composition concentration, reliably higher at 0.1 mg than at 0.05 mg.

The pharmaceutical composition effect on the glutamate toxicity model background compared with the physiologic effect without glutamate toxicity showed itself in:

- lower aspartate extracellular level;

- significant, dose dependent augmentation of GABA level;

- stabilization of glycin and taurin concentrations; and

- trend to glutamate level decrease.

The pharmaceutical composition pharmacologic effect (higher granule cells survival rate in cerebellar cells culture under glutamate cytotoxic effect) is due to the drug regulatory effect on the excitatory and inhibitory mediations levels, ensuring the necessary protection of the neurons against damage. The most interesting effect of the pharmaceutical composition action on the amino acids extracellular pool change is, in addition to the GABA concentration growth, the taurin level augmentation. This amino acid, being SH-groups donator, increases the glutathione-peroxidase system effect, improving thus the neurons cellular membranes antioxidant protection. The used glutamate toxicity model allows to assess the pharmaceutical composition effect on the dynamics of both the neurotransmitters extracellular level, including the amino acids metabolic pool, and the synaptic pool (indirectly). In other words, the pharmaceutical composition neuroprotective effect is explained by the glutamate level stabilization, with the trend to reduction due to both the neurotransmission inhibitory component activation and the excitatory neurotransmitters spillover decrease.

As promised at the beginning of this description, the Applicant gives below the more detailed information concerning the biologically active complex used in the claimed Invention.

The claimed method of production of the biologically active protein-polypeptide complex is characterized in that the quick-frozen mid first third to mid last third gestational age hoof livestock embryonic brain is unfrozen, and, gradually, within the 2°C to 28°C temperature range:

- homogenized in buffer solution, simultaneously extracting in presence of the buffer solution proteolysis reversible inhibitors and nonionic detergents at pH min. 5.2 and max. 8.5, at the solution / tissue volume ratios min. 1:0.5;

- the homogenate is separated from the undissolved tissular and cellular components by centrifuging at 10,000 g to 30,000 g during 90 to 30 minutes, respectively;

- the supernatant is stepwise filtered down to the max. 0.45 $\mu$m mesh filters;

- the filtrate undergoes the anion-exchange chromatography using a saline buffer solution as mobile phase and separating the linked to sorbent proteins and polypeptides in stepwise gradient, with the help of a 0.08 to 0.26 mol/l ionic strength and 5.2 to 8.5 pH eluent, increasing the mobile phase ionic strength in the 0.02 mol/l steps, and beginning to collect the target fractions with the min. 0.1 ionic strength solution; and

- the collected target fractions are desalinized in dialysis or gel filtration, and, having added the bacteriostatic and fungicidal preservatives, 0.06 mg/ml max. total concentration, and solubilizer, 0.01 mg/ml max. total concentration, are ultra filtered at max. 0.22 $\mu$m mesh, and then sterile packed.

The other peculiarity is the biologically active protein-polypeptide complex itself, obtained by the abovementioned method; this complex produces the tissue-specific reparative effect on the nervous tissue; it contains the negatively charged faintly acid neutral proteins and polypeptides relating to the growth factor, differentiation factor, signaling molecules, that determine its biologic and pharmacologic activity, with the 5 to 200 kDa molecular masses, wherein at least 80% of the total protein mass has the 10 to 120 kDa molecular mass, and wherein a peak exists at the 274-284 nm wave length in the UV-visible spectrum and bands exist in the pI 4.2 to 8.4 range at isoelectric focusing in a 5% polyacrylamide gel.

When separating the target product, at the homogenization and anion-exchange chromatography stages, the buffer media pH must be 5.2 to 8.5; the buffer solution must, at the homogenization and extraction stages, have the sufficient concentrations of detergents and proteolysis inhibitors; the anion-exchange chromatography stage must use, as mobile phase, the solutions of the 0.08 to 0.26 mol/l 0.02 mol/l step ionic strength, pH must be 5.2 to 8.5, and the target fractions collection must start with the min. 0.1 ionic strength eluent; the resulting target fractions must contain the 5 kDa to 200 kDa molecular mass proteins and the complex must contain min. 80% of total protein 10 kDa to 120 kDa molecular mass proteins and polypeptides; the resulting complex must be characterized by a peak at the 274-284 nm wave length in the UV-visible spectrum and bands in the pI 4.2 to 8.4 range at isoelectric focusing in a 5% polyacrylamide gel and the proteolytic proteins composing it must have proteolytic activity; and the feedstock must be the quick-frozen mid first third to mid last third gestational age hoof livestock embryonic brain. With other parameters, the complex protein-polypeptide composition and concentration ratios of the forming biologically active components will differ.

As noted above, the extracted biologically active substance, being a protein-polypeptide complex, differs from the prior ones extracted from the animal brain, as of the feedstock  source at the fixed gestational ages, as of the molecular masses and qualitative composition of the protein-polypeptide fractions, as of the evolutionarily fixed and in ontogenesis growth proteins, differentiation factors, and signaling molecules concentration ratios, and as of the biological and pharmacological activity and this activity specificity. The anion-exchange chromatography extracted protein-polypeptide complex that allows to obtain the eluent ionic strength dependent charge level target fractions consists of the weak-acid and neutral protein and polypeptide components due to the described above empirically combined conditions. A peculiarity of the obtained complex is the existence of the large enough (up to 200 kDa) no allergenicity and no immunotoxicity protein molecules; this is due, first of all, to the feedstock (quick-frozen embryonic brain at certain gestation ages) organo-specific characteristics. The protein-polypeptide complex feedstock characteristics condition both the availability of the components required for the whole range of biologic activities, i.e., growth factors, tissues and cells differentiation factors, and signaling molecules, and their concentration ratios, as well. Moreover, despite that at different gestational stages the protein-polypeptide composition qualitative composition of the embryonic brain (feedstock) changes, with the claimed method, the target components remaining within the evolutionarily fixed concentration ratios ranges, ensure the required biological activity of the substance. The claimed protein-peptide complex feedstock and production parameters characteristics variations do both deteriorate its biological activity and may lead to adverse events, such as the carcinogenicity, allergenicity, immunotoxicity, etc. The freeze drying of the claimed biologically active substance also significantly (up to 70%) deteriorates its biologic tissue- and organo-specific activity.

The following examples illustrate the production method.

**Example 16. Biologically active complex production method.**

Protein fraction production from ovine embryonic brain. 200 grams of quick-frozen ovine embryonic brain, 16 to 18 weeks gestational age, is unfrozen and 5-minute homogenized in 1000 ml of 0.05 M TRIS-glycin-phosphate buffer, pH 8.0, containing 1 mM of ethylenediaminotetraacetic acid (EDTA) and 0.1% mannitol, at 4°•. The homogenate is filtered through a dense cloth to separate the ballast substances, then 60-minute centrifuged at 20, 000 g, then filtered through a 0.45 $\mu$m mesh membrane filter at 4°•. The filtrate is then applied to a 200 ml volume chromatographic column with the Toyopearl DEAE-650M anion-exchange medium; the column is equilibrated with 4 volumes of the glycin-phosphate buffer, pH 8.0, containing 0.1 mM of EDTA. The proteins linked to the carrier are separated by the stepwise gradient of the 0.05 M glycin-phosphate buffer, pH 8.0, containing 0.04 M of KCl, 0.02 M-step increasing the salt concentration; the target fractions collection starts at the 0.06 M salt concentration. Chromatography temperature: 2 to 4°C. The target fraction undergoes the stage by stage filtering at max. 8.0 kfg/cm$^2$ back pressure, through 5 kDa and 200 kDa retention potential materials; then it is desalinized, and diluted with the 0.05 M glycin-NaOH solution, pH 7.4, down to the 1.2 mg/ml protein concentration, adding the polyoxyethylenesorbite monooleate (Tween 20) to the 0.01 mg/ml total concentration. The solution is sterilizing filtered, through a max. 0.22 $\mu$m mesh membrane filter. To characterize the resulting protein-polypeptide fraction the ultraviolet spectrophotometry, gel chromatography, electrophoresis in polyacrylamide gel, and amino acid analysis were used. The solution ultraviolet spectrum is read in the 250 to 350 nm wave length range, the maximum absorption is observed at 280$\pm$5 nm. To determine the molecular mass of the complex forming proteins and polypeptides the following methods are used: gel chromatography with Superdex 75 (GE Healthcare) and denaturing SDS-Page electrophoresis in 12-percent polyacrylamide gel in comparison with a standard marker proteins set. The column is calibrated with the protein standards, molecular masses range: 13 kDa to 250 kDa. The above methods have found that the drug contains the 5 kDa to 200 kDa proteins and polypeptides, of which 82% have the molecular mass within the 10 to 120 kDa range. Amino acid analysis (%) : Asp: 10.82 + 2.5; Thr: 5.4 + 1.2; Ser: 5.2 + 1.3; Glu: 16.2 + 3.1; Pro: 7.045 + 2.4; Gly: 5.2 + 2.2; Ala: 5.4 + 1.2; Val: 7.08 + 2.7; Met: 2.65 + 1.3; He: 4.45 + 1.5; Leu: 9.4 + 2.2; Tyr: 4.02 + 1.1; Phe: 4.8 + 1.3; Orn: 0.48 + 0.1; Lys: 8.48 + 2.1; His: 2.8 + 0.8; and Arg: 6.52 + 2.1.

**Example 17. Biologically active complex production method.**

Protein fraction production from the piglet porcine embryonic brain. 200 grams of quick-frozen porcine embryonic brain, 3 to 4 weeks gestational age, are unfrozen and 5-minute homogenized in 800 ml of 50 mM TRIS-glycin-phosphate buffer, pH 5.8, containing 1 mM of ethylenediaminotetraacetic acid (EDTA) and 0.1% maltose, at 4°•. The homogenate is filtered through a dense cloth to separate the ballast substances, then it is 30-minute centrifuged at 30, 000 g, and then filtered through a 0.45 $\mu$m mesh membrane filter at 25°•. The filtrate is then applied to a 250 ml volume chromatographic column with the DEAE-Sepharose anion-exchange medium; the column is equilibrated with 4 volumes of the maleate buffer solution, pH 5.8, containing 0.1 mM EDTA. The proteins linked to the carrier are separated by the stepwise gradient of the maleate buffer solution, pH 5.8, containing 0.04 M NaCl, 0.02 M-step increasing the salt concentration; the target fractions collection starts at the 0.06 M salt concentration. Chromatography temperature: 2 to 4°C. The target fraction is stage by stage ultra filtered at max. 8.0 kfg/cm$^2$ back pressure, through 5 kDa and 250 kDa retention potential materials, then desalinized, and diluted with the 50 M aspartate-NaOH solution, pH 7.4, to the 1.0 mg/ml protein, adding the polyoxyethylenesorbite monooleate (Tween 20) to the 0.005 mg/ml total concentration. The solution is sterilizing filtered, through a max. 0.22 $\mu$m mesh membrane filter. To characterize the resulting protein-polypeptide fraction the ultraviolet spectrophotometry, gel chromatography, electrophoresis in polyacrylamide gel, and amino acid analysis were used. The solution ultraviolet spectrum is read in the 250 to 350 nm wave length range, the maximum absorption is observed at 280$\pm$5 nm. To determine the molecular mass of the complex forming proteins and polypeptides the following methods are used: gel chromatography with Superdex 75 (GE Healthcare) and denaturing SDS-Page electrophoresis in 12-percent polyacrylamide gel in comparison with a standard marker proteins set. The column is calibrated with the protein standards, molecular masses range: 13 kDa to 250 kDa. The above methods have found that the drug contains the 5 kDa to 200 kDa proteins and polypeptides, of which 82% have the molecular mass within the 10 to 120 kDa range. Resulting complex amino acid analysis (%) : Asp: 10.82 + 1.3; Thr: 5.4 + 0.9; Ser: 5.2 + 1.1; Glu: 16.2 + 1.9; Pro: 7.045 + 1.7; Gly: 5.2 + 0.8; Ala: 5.4 + 1.1; Val: 7.08 + 2.3; Met: 2.65 + 0.6; He: 4.45 + 0.8; Leu: 9.4 + 2.5; Tyr: 4.02 + 0.6; Phe: 4.8 + 1.1; Orn: 0.48 + 0.1; Lys: 8.48 + 2.3; His: 2.8 + 0.7; Arg: 6.52 + 2.1.

**Example 18. Effect of biologically active complex (drug) on brain explants development**

The experiments used 52 fragments of the brain cortex and 40 fragments of the cerebrospinal ganglions of the 10- to 12-day gallinacean embryos. The nutrient medium to cultivate the explants contained: Eagle's solution: 35%, bovine fetal serum: 25%, Hanks' solution: 35%, and gallinacean embryonic extract: 5%; glucose (0.6%), insulin (0.5 U/ml), penicillin (100 U/ml), and glutamine (2 mM) were added. The brain cortex and cerebrospinal ganglions fragments were placed in this medium and cultivated 2 days on collagenic substrate, in Petri dishes, in thermostat, at 36.7°C. The protein-polypeptide complex under study and the 0.5, 1, 2, 10, 20, 50, 100, 200, 400, 800, and 1000 ng/ml Cerebrolysin and Cortexin as positive control were added to the experimental medium. The area index, i.e., the total explant with the growth zone area to the brain fragment outgoing area ratio served as biological activity criterion. The difference reliability of the compared mean area indexes was evaluated using the Student's t-criterion. The area index was expressed in percents; the control area index was assumed 100%. The growth zone of the control brain explants was formed by the short neurites, glia progenitor cells, and fibroblast-like cells. The following experimental series were set up when studying the direct effect of the drug on the brain fragments. Cerebrolysin and Cortexin in various concentrations were added to the gallinacean embryos brain cortex and cerebrospinal ganglia explants nutrient medium. On the 3rd day of cultivation with the 100 ng/ml Cerebrolysin addition the reliable brain cortex explants area index augmentation by $24\pm3.5\%$ was observed, compared with the control area indexes. No reliable brain cortex explants area indexes were observed at other Cerebrolysin concentrations. On the 3rd day of cultivation with the 100 ng/ml Cerebrolysin addition the reliable brain cortex explants area index augmentation by $24\pm3.5\%$ was observed, compared with the control area indexes. A reliable brain cortex explants area index augmentation was observed: by 28+3.5% at 100 ng/ml Cortexin, and by 23+2% at 200 ng/ml Cortexin. No reliable brain cortex explants area indexes were observed at other Cerebrolysin concentrations. A distinct brain cortex explants development activation under the 20 ng/ml complex under study was observed: the experimental explants area index was reliably by $48\pm4.5\%$ greater than that of the control explants and comparison explants under the Cortexin and Cerebrolysin. No reliable explants area index augmentation was observed when adding the Cerebrolysin into the cerebrospinal ganglions explants cultivation medium; with the Cortexin, the explants area index augmentation by $22\pm3\%$ was observed. With the complex under study addition into the cerebrospinal ganglions explants cultivation medium the explants area index augmentation by $36\pm3.5\%$ was observed. When studying the longer, 7-day, brain cortex and cerebrospinal ganglions explants cultivation, the same neurite-stimulating effects were observed with the same complex under study concentrations. Thus, as of the brain tissues, the lower efficient protein-polypeptide complex under study concentrations threshold was observed compared with the Cerebrolysin and Cortexin. Thus, the brain cortex explants fragments growth zone augmentation was observed only for the 100 ng/ml Cerebrolysin and 50 and 100 ng/ml Cortexin; for the protein-polypeptide complex under study the respective concentrations were 20, 10, 50, and 100 ng/ml. That said, the protein-polypeptide complex stimulating effect intensity was reliably higher than for the Cortexin and Cerebrolysin. No reliable Cerebrolysin effect on the cerebrospinal ganglions explants growth zone augmentation was observed; the Cortexin reliably increased the area index by 24%, while the protein-polypeptide complex under study provoked the area index augmentation by 36%. This shows the well expressed and directed stimulating effect of the drug on different brain divisions neurons.

**Example 19. Biologically active complex toxicity study.**

The protein-polypeptide complex systemic toxic effects were studied in the acute toxicity conditions, single administration, and chronic toxicity, long-term administration [5]. The acute toxicity study used 72 outbred male white mice, 20 to 22 grams body weight. The animals were randomized into 6 equal groups. The animals received one fold intramuscular injections of 5 mg/kg, 10 mg/kg, 100 mg/kg, 200 mg/kg, and 500 mg/kg complex in 0.5 ml of sterile 0.05 M glycin-NaOH •• 7.5 solution. The control group animals received the same doses of the sterile 0.05 M glycin-NaOH •• 7.5 solution. The subacute toxicity study used 95 outbred male white rats, 150 to 180 grams body weight. The experimental animals received the protein-polypeptide complex during 90 days, once daily, intramuscularly, 1 mg/kg, 10 mg/kg, 50 mg/kg, and 100 mg/kg in 0.5 ml of sterile 0.05 M glycin-NaOH •• 7.5 solution. The control group animals received the same doses of the sterile 0.05 M glycin-NaOH •• 7.5 solution. The peripheral blood morphologic composition and properties were studied before the complex introduction and on the 30th, 60th, and 90th days. Upon the experiment completion, the blood biochemistry and coagulology were measured.

The chronic toxicity study used 112 male guinea pigs, 250 to 680 grams body weight, during 6 months. The experimental animals received the protein-polypeptide complex during 6 months, once daily, intramuscularly, 1 mg/kg, 10 mg/kg, 50 mg/kg, and 100 mg/kg in 0.5 ml of sterile 0.05 M glycin-NaOH •• 7.5 solution. The control group animals received the same doses of the sterile 0.05 M glycin-NaOH •• 7.5 solution. The erythrocytes, hemoglobin, reticulocytes, thrombocytes, and leucocytes numbers, leucocyte formula, erythrocyte sedimentation rate (ESR), and erythrocyte resistance were determined in the animals' peripheral blood using conventional methods. In addition, the total protein in blood serum (Lowry's method), and potassium and sodium (plasma spectophotometry) were determined. Upon completion of the experiment the pathomorphological exams of the brain and spinal marrow, spinal ganglions, thyroid and parathyroid glands, adrenal glands, seminal glands, hypophysis, heart, lungs, aorta, liver, kidneys, urinary bladder, pancreatic gland, stomach, small bowel, large bowel, thymus, spleen, lymphatic glands, and bone marrow were performed. The acute toxicity studies revealed that the single administration of the complex under study in animals, in the dose exceeding the therapeutic one recommended for clinical use more than 5000-fold does not provoke toxic reactions; this means a large therapeutic range of the complex.

The complex subacute and chronic toxicities studies revealed no side effects at the long term administration in the doses exceeding the supposed therapeutic one 3000-fold. The exam of the complex effect on the guinea pigs peripheral blood morphology and biochemistry 3 and 6 months after the study start revealed no reliable alteration of these parameters.

No pathological changes were revealed when assessing the animals' performance status, peripheral blood morphology and biochemistry, internals, cardio-vascular and respiratory system, and liver and kidneys functions. Hence, the protein-polypeptide complex obtained by the claimed method does not have, at long term administration to animals, any toxic properties preventing from its use as a parenteral drug.

**Example 20. Substance mutagenicity.**

To assess the claimed protein-polypeptide complex mutagenicity, the following set of tests was used:

- Ames Salmonella/microsome gene mutagenicity assay using the TA 97, TA 98, and TA 100 Salmonella typhimurium strains as test objects; and
- micronuclei in murine bone marrow cells.

A protein-polypeptide complex sample was tested, in form of transparent liquid sterile packed in a dark glass vial, 20 ml, 2.3 mg/ml substance.

**Ames Test Protein-Polypeptide Complex Mutagenicity Assessment.**

The Salmonella typhimurium mutagenicity assay is a bacterial test system to account for the histidine prototrophy gene mutations under effect of the chemical compounds and/or their metabolites, inducing the mutations of the base substitution or reading frame shift in organism genome type. The mutagens inducing the base pairs substitution are the agents provoking the base pairs substitution mutations in the DNA molecule. The mutagens inducing the genetic code reading frame shift mutations are the agents provoking the addition or deficit of single or multiple base pairs in the DNA molecule.

The method's purpose is to reveal the capability of the pharmaceutical substances or their metabolites to induce the gene mutations in the Salmonella typhimurium indicator strains. The bacteria are treated with a compound under test with (SM+) or without (SM-) metabolic activation system. After a certain time incubation, the different test strains revertant colonies number is compared with that of the negative control spontaneous revertant (untreated or solvent treated cultures). If the compound under test and/or its metabolites have mutagenic activity, they will induce the reverse mutations from histidine auxotrophy to prototrophy in the Salmonella typhimurium histidine-dependent strains.

The tests used a set of the Salmonella typhimurium indicator strains allowing to register the genetic code reading frame shift (TA 98 and TA 97) base pair substitution (TA 100) mutations. The strains originated from the Russian National Collection of Industrial Microorganisms, Research Institute for Genetics and Selection of Industrial Microorganisms, Scientific Center of Russian Federation.

The bacterial cultures handling Rules of procedure, the strains genotype checks, their museum keeping rules, the experimental utensils, reagents, nutrient media and solutions, rat liver homogenate getting, activating mix compounding, and statistical analysis methods complied with the standards described in detail in the relevant literature.

The metabolic activation used the S9 fraction of the Wistar male rats liver; 5 days before sacrifice the rats received the sovol microsomal enzymes inductor (300 mg/kg, single, intraperitoneally). To control the metabolic activation system the ethidium bromide (10 $\mu$g/dish), TA 98 strain, SM+, was used.

The 2.3 mg/ml protein sterile protein-polypeptide complex solution was examined: 0.3 and 0.1 ml/dish mother solution and three 10X dilution in physiological solution (0.1 ml/dish). The substance test doses were 690, 230, 23, 2.3, and 0.23 $\mu$g/dish.

The experiment had positive controls; for them the mutation-inducing substances in relevant microorganisms strains with or without activation were used. The no-activation variants used the sodium azide, 10 $\mu$g/dish, TA 100 strain, SM-; 2, 7-diamino-4, 9-dioxy-5, 10-dioxo-4, 5, 9, 10-tetrahydro-4, 9-diazopyrene (DDDTDP), 10 $\mu$g/dish, TA 98 strain, SM-; and 9-aminocaridine (9AA), 50 $\mu$g/dish, TA 97 strain, SM-. To control the metabolic activation system activity the ethidium bromide (10 $\mu$g/dish), TA 98 strain, SM+, was used. The negative control used the physiological solution (0.1 ml/dish).

The selective semi-enriched agar (0.7%) in test tubes was melted in water bath at 100°C; then it was placed into the 45-46°C thermostatted water bath.

First, 0.1 ml of the relevantly diluted sample, then 0.1 ml of the bacterial suspension and 0.5 ml of the microsomal activating mix (metabolic activation variant) were put into the agar test tubes. Then the tube content was quickly blended and discharged onto the lower minimal agar in the Petri dishes. The microsomal activating mix and semi-liquid agar reserve introduction time per dish did not exceed 10 to 15 seconds. The dishes were left for 30 to 40 minutes at ambient temperature; after the first agar gelation they were put into a 37°• thermostat. The results were accounted after a 48-hour incubation.

The no metabolic activation system (SM-) and metabolic activation system (SM+) variants were experimented in parallel. In the SM- variant the direct mutagens (drugs mutagenic due to the compound initial structure activity) effect may be observed. As of the effect of the promutagens, i.e., the compounds whose effect associates with the mutagenic metabolites formation, this may be accounted when comparing the results of the SM- and SM+ compounds tests analysis data. Every control and experimental variants used 2 dishes each. The mutagenic effect was considered significative when the mean number of the revertants colonies per dish in experiment exceeded the control one 2- and more fold. The experimental results were taken into account at the condition of standard response in all the positive and negative control variants. The revertants colonies numbers in the solvent control, SM- and SM+ variants, were within the spontaneous level fluctuations range for the given strains. The strains response to the standard mutagens was within the common levels range.

The examined with all concentrations protein-polypeptide complex did not show any mutagenic effect for the TA 100, TA 98, and TA 97 strains, with and without metabolic activation system.

CONCLUSION: the protein-polypeptide complex is unable to induce gene mutations with the Salmonella typhimurium test strains within all the experimental concentrations range.

**Micronuclear Test Protein-Polypeptide Complex Mutagenicity Assessment in Mammalian Cells.**

The cytogenetic activity is the capacity of a substance to provoke structural and quantitative chromosomal abnormalities in the somatic and embryonic cells.

The micronuclei are the small size DNA-containing formations consisting of the chromosomal acentric fragments or lagged in ana-telophase chromosomes. At the telophase stage these fragments may get into the daughter cells nuclei or form individual or multiple micronuclei in cytoplasm.

The Study purpose is to reveal and quantitatively assess the cytogenetic activity (mutagenicity) of the pharmacologic drugs in the mammalian bone marrow polychromatophils. The method's base is the micronuclei cells microscopic registration.

The experimental used the male and female F1(CBAx C57BI6/J) hybrid mice, two-month age, 18 to 20 grams body weight. Every group included 6 animals. The animals were kept under the 12-hour light regime, with freely available water and feed. The single and five fold administration protein-polypeptide complex mutagenicity assessment animal experiments were in compliance with the official protocols. Three experimental series with relevant controls were performed: therapeutic dosage: males, single administration; subtoxic dosage: males, single administration; and therapeutic dosage: males and females, five fold administration.

Taking into account that the possible biologically active protein-polypeptide complex clinical administration method may be intravenous or endolumbar, this Study used the intraperitoneal one with mice. The protein-polypeptide complex mutagenicity assessment dose was defined on the basis of the maximum recommended daily therapeutic dose (TD) for human. The 2 ml at 0.5 mg/ml concentration IV administration is recommended. The therapeutic dose for human, including infant, may be 0.05 mg/kg/day. To calculate the mice experimental dose, the conversion factor is recommended to take into account the human and murine body surface area to body weight ratio. In our study it was 8.33. Hence, for the murine therapeutic dose the approximately 8.33 human therapeutic dose was taken (0.42 mg/kg).

For the subtoxic dosage the maximum possible experimental one was used, 23 mg/kg, as the substance concentration was 2.3 mg/ml; the conventional total substance administered to mice is 0.1 ml per 10 g body weight. Hence, recalculated for man, the maximum tested dose was 2.76 mg/kg.

Seven groups of animals, four experimental and three control ones, were formed (see Table 2). The 0.42 mg/kg substance was five times, at 24-hour intervals, administered to both the males and females. In all the groups the animals were sacrificed 6 hours after the last protein-polypeptide complex administration.

In two other experimental groups, the protein-polypeptide complex was single administered to the males, at 0.42 mg/kg and 23 mg/kg.

For the negative control the physiological solution was used. It was administered intraperitoneally to the males and females of the two control groups, the times and volumes were the same as for the experimental. Positive control: 20 mg/kg cyclophosphamide dissolved ex tempore in physiological solution, single intraperitoneal administration, 24 hours before sacrifice.

The bone marrow cells preparations for the micronuclei account were made in conventional manner, in accordance with the "Micronuclear Test Environmental Factors Mutagenicity Assessment in Mammalian Organs Cells" Guidelines. The sacrifice method was the cervical dislocation 6 hours after last drug administration. Both femoral bones were extracted, muscles were then removed. To wash out the bone marrow the bovine embryonic serum (NPP PanECO). The bone marrow from the both femoral bones was extracted into a microtube with a syringe. The suspension was centrifuged at 1000 rpm, 5 minutes; the supernatant was removed, the sediment was carefully resuspended. A drop of the suspension was used to prepare the smear then dried in air. The smear was then methanol fixed during 3 minutes. For staining the Romanowsky-Giemsa method was used. 2000 polychromatophils per animal were then analyzed with coded preparations. At the 500 erythrocytes count the polychromatophil to normochromatic erythrocytes ratio was determined. Analysis finished, the preparations were coded.

The positive result criterion is the reproducible and statistically significant growth of the micronuclei polychromatophil erythrocytes number in at least one experimental group compared with the control one. The positive result indicates that the substance induces the chromosomal damages and/or cells mitotic apparatus damages in experimental animals.

The micronuclei account results statistical treatment used the X2 criterion experimental to control series comparison; the polychromatophils proportion intergroup comparison used the Mann-Whitney criterion.

Mice bone marrow micronuclei polychromatophils account results: in no experimental variants the protein-polypeptide complex induced the mice bone marrow micronuclei polychromatophils proportion augmentation as compared between the experimental and relevant control series. The 20 mg/kg cyclophosphamide (positive control) intraperitoneally in male mice provoked 19.6-fold higher micronuclei cells count (62.1‰ vs. 3.16‰ in control, P < 0.001). The polychromatophil erythrocytes share in the total bone marrow erythrocytes was at approximately the same level for both the experimental and control groups; hence, no toxic effect of the substance in tested doses on hematogenesis. Under the positive control (cyclophosphamide) the polychromatophil to normochromatic erythrocytes ratio shift to the latter ones was recorded (compared with control, P < 0.005).

Thus, the micronuclei induction test in the mice bone marrow micronuclei polychromatophils at one and five fold 0.42 mg/kg body weight intraperitoneal administration to males and females (corresponding, as recounted, to the daily therapeutic dose for man) revealed no protein-polypeptide complex mutagenic activity. No mutagenic activity was revealed for the maximum possible, 23 mg/kg, single protein-polypeptide pharmaceutical composition administration in males. The protein-polypeptide complex showed no toxic effect as of the "polychromatophil erythrocytes proportion" parameter.

**Conclusion for the protein-polypeptide complex mutagenicity assessment**

The protein-polypeptide complex showed no mutagenic activity in either the Ames Salmonella/microsomes test or the mice bone marrow cells micronuclei induction test performed in accordance with the Guidelines for Experimental (Preclinical) Study of New Pharmacological Substances.

**Example 21. Substance specific activity [6, 7].**

**Local irritant effect.**

The studies showed that the biologically active protein-polypeptide complex substance has no local irritant effect when administered subcutaneously, intravenously, and intranasally. At the rapid intravenous administration the hyperthermia effects are possible. No local irritant effect on the nose and throat mucosa was revealed at the 1-month intranasal administration in rabbits.

**Immunotoxicity and allergenicity studies**

*Active cutaneous anaphylaxis*

The experimental used the guinea pigs, 10 per group, 3 groups. Sensibilization pattern: first injection subcutaneously; then two intramuscularly, alternate days, femoral area. On the 14th day, the substance was administered intracutaneously, in two fold dilution, on sheared areas of the back. To control the cutaneous sensibility, the physiological solution was administered to a same animal, on another sheared skin area. The control animals were administered an injection boosting substance (equal to the sensibilizing dose). Then the animals were intravenously administered 0.5 ml 1% Evans blue solution. Thirty minutes later the animals were sacrificed with ester; the blue spot on inner skin side at the administration point size was determined. The reaction was considered positive at the larger than 6 mm spot vs. max. 3 mm in control. In 3 cases the positive reaction was recorded in the group having received a 10X therapeutic dose. The number of reactions means the possibility of individual responses.

*Delayed type hypersensitivity reaction in mice.*

The experimental used the nonlinear, 18 to 20 g body weight mice, 10 animals per group. The experimental used 30 animals in total. The sensibilization was single, intracutaneously, at tailset, drug emulsion in NAF (1:1). The dose administered was 60 μl. The control animals were sensibilized with the PAF emulsion with Hanks' solution (1:1). To reveal the sensibilization, 5 days after, the mice were administered 40 μl of the test drug in Hanks' solution into a hind foot pad. Twenty four hours after test, the edema size was measured using the MK-0-25 engineering micrometer. The results statistical treatment did not reveal any significant difference in the feet thickness between the experimental and control groups (P > 0.05). The substance administration does not provoke the delayed type hypersensitivity reaction.

*Peritoneal macrophages phagocytic activity study.*

The experimental used the 180 to 200 g body weight albino rats, 30 animals, 10 per group. The two drug doses, therapeutic and 10-fold therapeutic ones, were studied; the third group was for control. The method is based on determining the lysing solution optical density after destruction of the phagocytes having absorbed the neutral red particles. The rats exposed to the drug under study were decapitated. The animals were intraperitoneally administered 5 ml of the 199 medium containing 20% of the bovine embryonic serum. Having massaged the peritoneal cavity, the medium was sucked out with a syringe. The portions of the cellular suspension obtained from the whole group of animals were joined in a common pool. The living cells concentration was brought to $1.5 \times 10^3$ cells/ml. The cellular suspension was then sterilely dispensed into the 40 mm diameter plastic Petri dishes, 2 ml each. The dishes were 2-hour incubated at 37°C. The supernatant with unstuck cells was disposed; the monolayer fixed to the plastic was twice washed with the 199 medium. A solution with the neutral red was put into the slightly dried dishes; these were then cured 1 hour at 37°C. The solution was poured off. The cells were washed with the 199 medium. Three milliliters of the lysing solution was put into every dish. The cells were thoroughly treated by rotating motion. The solution was poured into test tubes; the optical density was checked with spectrophotometer at 540 nm wave length.

The substance administration does not inhibit the macrophages activity. No statistically valid differences exist between the experimental and control groups results.

*Mast cells degranulation reaction*

The experimental used 30 female rats 180 to 200 g body weight. The substance solution was administered intraperitoneally in the therapeutic and 10-fold therapeutic doses; the intact group served as control. To obtain the mast cells suspension, the animals were decapitated, a buffer was injected into the peritoneal cavity; having massaged the anterior abdominal wall, the mast cells suspension was collected in centrifugal test tubes. The preparations were made on the glass slides stained with the 0.3% neutral red in alcohol solution. To 0.03 ml of the mast cells suspension, 0.03 ml of the experimental animal serum and 0.03 ml of the drug under study in 1:100 dilution were added. In control, the maximum degranulation was 5%. The preparations were covered with cover glass sealing the edges with wax. Then they were 15-minute incubated in thermostat at 37°C. The preparations were studied under microscope at X40, counting the degranulated and normal mast cells, 100 in total. The reaction was considered positive with the degranulation above 20%.

The substance in the 0.01 mg/kg concentration does not provoke the mast cells degranulation, is the same for the control; the 5% threshold was not overpassed. The ten fold therapeutic dose group reaction is feebly positive, however, within the statistical errors percentage.

**Prion tests.**

The assays were taken from 4 vials. The reaction used the enzyme-linked immunosorbent assay method, with a 96-socket polystyrene pad. Diagnostic test system: PrionScreen. Spectrophotometer: Digiscan. Every assay underwent 4 repeats. Controls: K-: 8 sockets, K+: 8 sockets. The resulting control and study optical densities were consistent with the reaction conditions.

Results: no prions revealed in substance under study.

**General conclusion.**

The biologically active protein-polypeptide complex original substance toxicity in the acute and subchronic experiments, and its local irritant, immunotoxic, and allergenic effects were studied; and search for possible prion infections was conducted.

The drug acute toxicity was experimentally studied with the BALB/C line mice. The drug was administered subcutaneously, intraperitoneally, and per os. The maximum possible for administration in mice dose is 0.2 ml, i.e., 10 mg/kg. It was revealed that in the specified dose the drug administered by any of the three methods did not result in the animals' death or provoke acute poisoning.

The subchronic experiment used the Wistar line rats, both mature and immature. The drug under study was administered subcutaneously, 0.01 mg/kg therapeutic and 0.1 mg/kg 10X therapeutic dosages; and intravenously, 0.0025 mg/kg, and x 10 0.025 mg/kg. The intranasal experiment, 1 shot/day, i.e., 1.5 mg/ml, 0.2 ml, used the Chinchilla rabbits.

The toxicity study used the conventional methods for the blood hematology and biochemistry, and viscera histology.

It was revealed that the substance administration by any way, in both the therapeutic and 10X therapeutic doses, in the course of 1 month, provokes no viscera alteration and has no toxic effect on the blood system. No local irritant effects were revealed at any administration.

The allergenicity studies used the intracutaneous applications in guinea pigs. The substance did not provoke any allergic response in therapeutic doses. For the ten fold therapeutic dosage, three cases of hypersensitivity were recorded. No immunotoxicity with the Wistar rats was revealed.

The prion infections search used the enzyme-linked immunosorbent assay method. The drug is free of these causative agents.

**Example 22. Effect on cell population cytokinetic parameters.**

**Materials and Methods**. The 3T3 murine fibroblasts were cultivated on the DMEM (PanEco, Moscow, Russia), with additions: 584 mg/l glutamine; antifungal-antimicrobial agent (Sigma, USA) according to manufacturer's instruction; and 10% vol. standardized bovine embryonic serum (HyClone, USA). For experimental, the cells were planted onto the 24-socket pads (Corning), 3000 cells/ml (socket). For morphology the cells were planted onto the 12 mm diameter cover glasses (Ted Pella, USA), placed in the sockets.

*Test substances application.* A cell culture having reached the required density, the culture medium was replaced with the fresh one of the same composition (control); DMEM without serum with 2 mg/ml of BSA (Sigma) medium (negative control); DMEM without serum with 10% vol. biologically active substance (experiment 1); and DMEM with 10% serum and 10% biologically active substance (experiment 2).

**Experimental procedures.** The cells were analyzed 8, 24, and 48 hours after the test agents application. The culture in vivo state was analyzed; the cells photos were taken: Axiovert 200M phase-contrast inverted microscope (Carl Zeiss, Germany), with EC Plan-Nefluar 10x NA 0.5 field lens, and ORCA II Erg-2 digital camera (Hamamatsu Photonics, Japan). To determine the cells number, the cells sheet was dissociated with the Versen solution (PanEco) and 0.025% trypsin solution (PanEco) 3:1 mix. The resulting suspension was centrifuged at 300 g; the sediment was resuspended in the Hanks' solution, and counted in the Goryaev chamber. To analyze the cell population cytokinetic parameters, the cells cultivated on the cover glasses were fixed with the 1% gluteraldehyde on 0.1 M phosphate buffer at 4°C; permeabilized with the 0.5% Triton X-100 solution on PSB (10 minutes); washed with the distilled water; stained with the Mayer's hematoxylin (90 seconds) (Sigma); desiccated; and put into DePeX. After permeabilization, the cells were stained with the DAPI (0.1 $\mu$g/ml) and put into Moviol. The resulting preparations were studied under the Axioskop II (Carl Zeiss) fluorescence microscope, Plan-Neofluar 40x NA 1, 2 and 100x NA 1, 3 field lenses.

**Results. Population cytokinetic parameters.**

*Control.* 8 hours. Mitotic index: 8%. Apoptosis and necrosis: 0%.

24 hours. Mitotic index: 11.9%. Apoptosis and necrosis: 0%. Cells in socket: 265,000.

48 hours. Mitotic index: 9%. Apoptosis and necrosis: 0%. Cells in socket: 780,000.

BSA. 8 hours. Mitotic index: 5%. Apoptosis and necrosis: 0%.

24 hours. Mitotic index: 5.5%. Apoptosis and necrosis: 0%. Cells in socket: 167,500.

48 hours. Mitotic index: 3%. Apoptosis and necrosis: 0%. Cells in socket: 625,000.

*Biologically active substance.*

8 hours. Mitotic index: 6%. Apoptosis and necrosis: 0%.

24 hours. Mitotic index: 7.1%. Apoptosis and necrosis: 0%. Cells in socket: 250,000.

48 hours. Mitotic index: 6%. Apoptosis and necrosis: 0%. Cells in socket: as counted: 187,000; actual:• 400,000 (ejection).

*Serum + Biologically active substance.*

8 hours. Mitotic index: 8%. Apoptosis and necrosis: 0%.

24 hours. Mitotic index: 4.5% (ejection). Apoptosis and necrosis: 0%. Cells in socket: 317,500.

48 hours. Mitotic index: 9%. Apoptosis and necrosis: 1%. Cells in socket: 680,000.

All the experimental lines show the biomass augmentation; no cytotoxic effect is observed in any case. In the negative control, the proliferation efficiency is minimal; with the protein-polypeptide complex the proliferation is stimulated compared with the negative control. The positive control and substance and serum added medium show a same proliferation level.

The cells incubated with the substance form thin and long processes. This is especially well seen in the first experimental day. With the serum on the substance background the effect remains.

**Conclusions:**

1) The biologically active substance consisting of the protein-polypeptide complex is not a cytotoxic agent.

2) The substance contains growth factors, but is not as efficient as proliferation stimulator as the blood serum.

3) The substance provokes the cells shape changes, with a well visible processes formation.

**Example 23. Effect on brain blood supply and rats survival rate under brain ischemic damage.**

The experimental used the Wistar male rats, 180 to 250 g body weight, under irreversible bilateral occlusion of the common carotid arteries, using the generally known methods [8-10]. All the veterinary manipulations were aseptic, to keep the experimental animals status. The experimental animals were kept in standard conditions [11].

The rats received the ether (chemically pure diethyl ether, OAO "Medhimprom") anesthesia; then a 8 to 10 cm long skin discission was made in the neck region, along the median line, to access the carotid arteries. The both carotid arteries were exposed, separating the vagus and sympathetic nerves. The silk threads (Silk blk 17 x 45 cm/• 3/USP2/ Non needled/ Ethicon Johnson & Johnson, cat. No W203) were pulled under the vessels; then, together with an assistant, the both carotid arteries were single-step ligated with triple friction knot. The skin discission was then sutured with the surgical silk (Silk blk 100 cm/• 5/Sgle armed KP-3USP2/ Non needled/ Ethicon Johnson & Johnson, cat. No W794), one or two surgical stitches, with further wound treatment with the 5% iodine tincture. The physiological solution or cellex were administered intraperitoneally. The total manipulations time was 8 to 10 minutes; then the animals were placed into cage.

The survival rate was followed up immediately after the procedure and in the course of the first day, i.e., the final survival rate was fixed in 24 hours. The drug biological activity in form of the brain protection under incomplete global ischemia at single-step carotid arteries ligation was assessed as follows:

- **expressed:** survival rate: > 80% rats;

- **median expressed:** survival rate: 70% to 80% rats; and

- **unexpressed:** survival rate: < 70% rats.

The data statistical processing used the Fischer exact test, accounting for the comparisons multiplicity.

**Experiment 1.**

Three groups of animals were used:

- **group 1:** control animals, physiological solution with sterile disposable insulin syringe: 0.5 ml/rat in 1 hour and 0.5 ml/rat in 5 hours after carotid arteries ligation;

- **group 2:** sham operated animals: same manipulations as in control but without carotid arteries ligation; physiological solution 0.5 ml/rat in 1 hour and 0.5 ml/rat in 5 hours; and

- **group 3:** animals after carotid arteries ligation; biologically active substance: 0.5 ml/rat 0.1% solution (0.2 mg/kg) in 1 hour and 0.5 ml/rat in 5 hours.

The experiments showed that the greatest number, i.e., 11 of 24, of the animals in control died within the 1st hour after the carotid arteries ligation; 4 more animals died within the next 24 hours; in total, 15 rats of 24 died (63%, see Table 1). Among the sham operated animals 2 (17%) rats died immediately after the operation. The biologically active substance 0.2 mg/kg (0.5 ml 0.1% solution) intraperitoneally in 1 and 5 hours provoked death in only two animals: 34/36 rats survived. The compared to control survival rate augmentation is 95%. Table 8 shows the obtained data.

**Table 8. Effect of biologically active substance on rats survival rate under ligation of both carotid arteries**

| Groups of animals | Expressed bioactivity | |
|---|---|---|
| | Survived | Died |
| Group 1 Control: 24 rats | 9 of 24 (37%) | 15 of 24 63% |
| Group 2 Sham operated 12 rats | 10 of 12* (83%) | 2 of 12 (17)% |
| Group 3 Substance 0.2 mg/kg 36 rats | 34 of 36* (95%) | 2 of 36 (5%) |
| * -P • 0.01 compared with control. | | |

Hence, the protein-polypeptide complex biologically active substance ensures a statistically significant animals survival rate augmentation compared with control under incomplete global ischemia of brain at single-step both carotid arteries ligation.

**Experiment 2.**

Three groups of animals were used:

- **group 1:** control animals, physiological solution with sterile disposable insulin syringe: 0.5 ml/rat in 1 hour and 0.5 ml/rat in 5 hours after carotid arteries ligation;

- **group 2:** animals after carotid arteries ligation; biologically active substance: 0.5 ml/rat 0.1% solution (0.2 mg/kg) in 1 hour and 0.5 ml/rat in 5 hours.

- **group 3:** animals after carotid arteries ligation; 0,2 mg/kg freeze-dried biologically active substance: 0.1 mg dry weight in 0.1% physiological solution, 0.5 ml/rat in 1 hour and 0.5 ml/rat in 5 hours.

The experiments showed that the greatest number, i.e., 11 of 24, of the animals in control died within the 1st hour after the carotid arteries ligation; 4 more animals died within the next 24 hours; in total, 15 rats of 24 died (63%, see Table 1). The diluted freeze-dried biologically active substance 0.2 mg/kg (0.5 ml 0.1% solution) intraperitoneally in 1 and 5 hours provoked death in only four animals: 8/12 rats survived. The biologically active substance 0.2 mg/kg (0.5 ml 0.1% solution) intraperitoneally in 1 and 5 hours provoked death in only two animals: 34/36 rats survived. The compared to control survival rate augmentation is 95%. Table 9 shows the obtained data.

**Table 9. Effect of biologically active substance on rats survival rate under ligation of both carotid arteries**

| Groups of animals | Expressed bioactivity | |
|---|---|---|
| | Survived | Died |
| Group 1<br>Control: 24 rats | 9 of 24<br>(37%) | 15 of 24<br>63% |
| Group 2<br>Dried cake<br>12 rats | 8 of 12*<br>(66,7%) | 4 of 12<br>(33,3)% |
| Group 3<br>Substance 0.2 mg/kg<br>36 rats | 34 of 36*<br>(95%) | 2 of 36<br>(5%) |
| * -P • 0.01 compared with control. | | |

Hence, the protein-polypeptide complex biologically active substance ensures a statistically significant animals survival rate augmentation compared with control under incomplete global ischemia of brain at single-step both carotid arteries ligation.

The protein-polypeptide complex freeze-dried fraction biological activity under incomplete global ischemia of brain at single-step both carotid arteries ligation is much lower than that of the liquid substance.

**Industrial Applicability**

The Invention is intended for use in the medicine and relates to the new method of treatment of ischemic and hemorrhagic character cerebral and spinal circulation accidents with use of the neuroprotective drugs.

**Claims**

1. Method of treatment of the ischemic and hemorrhagic character acute cerebral and spinal circulation accidents, wherein, from the first hours of the acute focal neurologic symptoms, the 0.05 to 0.8 mg/day pharmaceutical composition is administered subcutaneously, intramuscularly, intranasally, or intrathecally, which pharmaceutical composition has the tissue-specific reparative effect on the nervous tissue, and which pharmaceutical composition consists of the protein-polypeptide complex and pharmaceutically acceptable diluent, containing the officinal buffer solution and the excipients containing the high molecular weight compounds, stabilizers, preservatives, and solubilizers, which protein-polypeptide complex used in the pharmaceutical composition is obtained from the mid first third to mid last third gestational age livestock embryonic brain tissue, which complex contains the negatively charged faintly acid neutral proteins and polypeptides relating to the growth factor, differentiation factor, signaling molecules, and intercellular adhesion molecules, that determine its biologic and pharmacologic activity, with the 5 to 200 kDa molecular masses, wherein at least 80% of the total protein mass has the 10 to 120 kDa molecular mass, and **characterized by** a peak at the 274-284 nm wave length in the UV-visible spectrum and bands in the pI 4.2 to 8.4 range at isoelectric focusing in a 5% polyacrylamide gel.

2. Method of treatment of the acute cerebral and spinal circulation accidents claimed in 1, wherein, for the medium gravity patients the 0.1 to 0.2 mg (1 to 2 ml 0.1% solution) recommended dose pharmaceutical composition is used, administered subcutaneously or intramuscularly 1 to 2/day; or intranasally, 0.1% solution, 200 to 400 μl into every nasal passage, morning and day time, 7 to 10 days, renewing the treatment session in the early rehabilitation period, in 5 to 10 days;

3. Method claimed in 1, wherein, for the severe gravity as of the date of admission patients, the pharmaceutical composition is used, 0.2 to 0.6 mg daily dose (2 to 3 ml 0.1% solution, administered subcutaneously or intramuscularly 1 to 2/day; or by slow intravenous 0.2 to 0.4 mg (2 to 4 ml 0.1% solution) 1/day infusion, 5 to 10 days, renewing the treatment session in the early rehabilitation period, in 5 to 10 days;

4. Method claimed in 1, wherein, for the extreme condition as of the date of admission patient, with expressed impairment of consciousness the recommended drug dose is 0.4 to 0.8 mg, 0.2-0.4 mg (2 to 4 ml 0.1% solution), slow intravenous infusion, 1 or 2/day, 7 to 10 days; or intrathecally, 0.2 to 4 mg/day (2 to 4 ml 0.1% solution), once every 2 to 4 days, at no absolute counter indications to lumbar puncture; further on, intravenously or subcutaneously, relevant single or course doses, in function of the patient gravity.

## INTERNATIONAL SEARCH REPORT

| International application No. |
| --- |
| PCT/RU 2011/000327 |

A.  CLASSIFICATION OF SUBJECT MATTER

A61K 35/30 (2006.01), A61K 35/48 (2006.01), A61K 38/00 (2006.01), A61P 25/00 (2006.01), A61P 9/10 (2006.01)

According to International Patent Classification (IPC) or to both national classification and IPC

B.  FIELDS SEARCHED

Minimum documentation searched (classification system followed by classification symbols)

A61K 35/30, 35/48, 38/00, A61P 25/00, 9/10

Documentation searched other than minimum documentation to the extent that such documents are included in the fields searched

Electronic data base consulted during the international search (name of data base and, where practicable, search terms used)

DWPI, PatSearch, PubMed

C.  DOCUMENTS CONSIDERED TO BE RELEVANT

| Category* | Citation of document, with indication, where appropriate, of the relevant passages | Relevant to claim No. |
| --- | --- | --- |
| A | WO 2010/007620 A1 (DELAPHARM LTD.) 21.01.2010, the abstract, p. 14-16, p. 18, para. 3, p. 19, para. 3, p. 20, para. 2, p. 31-34, 74, п.п. 1, 8, 21, 27, 33-36, 38, 43, 45, 60 the claims, fig. 1 | 1-4 |
| A | SKOUPS P. Metodi ochistki belkov. "MIR", M., 1985, p. 312 | 1 |
| A | RU 2128511 C1 (OBSCHESTVO S OGRANICHENNOI OTVETSTVENNOSTIU "НИР") 10.04.1999, the abstract, the claims | 1-4 |
| A | RU 2275924 C2 (OBSCHESTVO S OGRANICHENNOI OTVETSTVENNOSTIU "GEROFARM") 10.05.2006, the abstract, the claims | 1-4 |

☐  Further documents are listed in the continuation of Box C.          ☐  See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 07 October 2011 (07.10.2011) | 02 November 2011 (02.11.2011) |

| Name and mailing address of the ISA/ | Authorized officer |
| --- | --- |
| **RU** | |
| Facsimile No. | Telephone No. |

Form PCT/ISA/210 (second sheet) (July 1998)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- UA 2128511
- RU 2104702
- WO 2049472 A
- US 939440 A

**Non-patent literature cited in the description**

- Encyclopedia of Drugs. 2006, 970
- Register of Medicinal Agents of Russia. Inpharm-chim, 1993, 935